(19) Europäisches Patentamt
European Patent Office
Office européen des brevets

(11) **EP 3 271 729 B1**

(12) **EUROPEAN PATENT SPECIFICATION**

(45) Date of publication and mention
of the grant of the patent:
**23.09.2020 Bulletin 2020/39**

(21) Application number: **16714298.3**

(22) Date of filing: **18.03.2016**

(51) Int Cl.:
**G01N 33/68** (2006.01)

(86) International application number:
**PCT/EP2016/056056**

(87) International publication number:
**WO 2016/146844 (22.09.2016 Gazette 2016/38)**

(54) **ASSAYS FOR RECOMBINANT EXPRESSION SYSTEMS**

ASSAYS FÜR REKOMBINANTE EXPRESSIONSSYSTEME

ESSAIS POUR DES SYSTÈMES D'EXPRESSION RECOMBINANTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.03.2015 EP 15159717**

(43) Date of publication of application:
**24.01.2018 Bulletin 2018/04**

(73) Proprietor: **Janssen Vaccines & Prevention B.V.
2333 CN Leiden (NL)**

(72) Inventor: **SCHOLTEN, Arjen
2333 CN Leiden (NL)**

(74) Representative: **Beslier, Victor et al
Janssen Vaccines & Prevention B.V.
Archimedesweg 4-6
2333 CN Leiden (NL)**

(56) References cited:
**EP-A1- 1 118 860     WO-A2-2014/134561**

• WILLIAMS T L ET AL: "Simultaneous quantification of hemagglutinin and neuraminidase of influenza virus using isotope dilution mass spectrometry", VACCINE, ELSEVIER LTD, GB, vol. 30, no. 14, 23 March 2012 (2012-03-23), pages 2475-2482, XP002725105, ISSN: 0264-410X, DOI: 10.1016/J.VACCINE.2011.12.056 [retrieved on 2011-12-22]
• MELKAMU GETIE-KEBTIE ET AL: "Label-free mass spectrometry-based quantification of hemagglutinin and neuraminidase in influenza virus preparations and vaccines", INFLUENZA AND OTHER RESPIRATORY VIRUSES, vol. 7, no. 4, 3 July 2013 (2013-07-03), pages 521-530, XP055120511, ISSN: 1750-2640, DOI: 10.1111/irv.12001
• WANDA I. SANTANA ET AL: "Quantification of Viral Proteins of the Avian H7 Subtype of Influenza Virus: An Isotope Dilution Mass Spectrometry Method Applicable for Producing more Rapid Vaccines in the Case of an Influenza Pandemic", ANALYTICAL CHEMISTRY, vol. 86, no. 9, 1 April 2014 (2014-04-01), pages 4088-4095, XP055211262, ISSN: 0003-2700, DOI: 10.1021/ac4040778

SEQUENCE LISTING

<110> Crucell Holland B.V.

<120> ASSAYS FOR RECOMBINANT EXPRESSION SYSTEMS

<130> P066005WO

<141> 2016-03-18

<150> EP 15159717.6
<151> 2015-03-18

<160> 27

<170> SeqWin2010, version 1.0

<210> 1
<211> 685
<212> PRT
<213> Artificial Sequence

<220>
<223> Env protein encoded by HIV vaccine drug product Ad26.HIV DP

<400> 1

Met Arg Val Thr Gly Ile Arg Lys Asn Tyr Gln His Leu Trp Arg Trp
1                   5                   10                  15

Gly Thr Met Leu Leu Gly Ile Leu Met Ile Cys Ser Ala Ala Gly Lys
            20                  25                  30

Leu Trp Val Thr Val Tyr Tyr Gly Val Pro Val Trp Lys Glu Ala Thr
            35                  40                  45

Thr Thr Leu Phe Cys Ala Ser Asp Ala Lys Ala Tyr Asp Thr Glu Val
    50                  55                  60

His Asn Val Trp Ala Thr His Ala Cys Val Pro Thr Asp Pro Asn Pro
65                  70                  75                  80

Gln Glu Val Val Leu Glu Asn Val Thr Glu Asn Phe Asn Met Trp Lys
            85                  90                  95

Asn Asn Met Val Glu Gln Met His Glu Asp Ile Ile Ser Leu Trp Asp
            100                 105                 110

Gln Ser Leu Lys Pro Cys Val Lys Leu Thr Pro Leu Cys Val Thr Leu
        115                 120                 125

Asn Cys Thr Asp Asp Val Arg Asn Val Thr Asn Asn Ala Thr Asn Thr
    130                 135                 140

Asn Ser Ser Trp Gly Glu Pro Met Glu Lys Gly Glu Ile Lys Asn Cys
145                 150                 155                 160

Ser Phe Asn Ile Thr Thr Ser Ile Arg Asn Lys Val Gln Lys Gln Tyr
            165                 170                 175

Ala Leu Phe Tyr Lys Leu Asp Val Val Pro Ile Asp Asn Asp Ser Asn
        180                 185                 190

Asn Thr Asn Tyr Arg Leu Ile Ser Cys Asn Thr Ser Val Ile Thr Gln

<pre>
          195                    200                    205
  Ala Cys Pro Lys Val Ser Phe Glu Pro Ile Pro Ile His Tyr Cys Ala
      210             215                 220

  Pro Ala Gly Phe Ala Ile Leu Lys Cys Asn Asp Lys Lys Phe Asn Gly
  225             230                 235                     240

  Thr Gly Pro Cys Thr Asn Val Ser Thr Val Gln Cys Thr His Gly Ile
              245                 250                     255

  Arg Pro Val Val Ser Thr Gln Leu Leu Leu Asn Gly Ser Leu Ala Glu
              260                 265                     270

  Glu Glu Val Val Ile Arg Ser Glu Asn Phe Thr Asn Asn Ala Lys Thr
              275                 280                     285

  Ile Met Val Gln Leu Asn Val Ser Val Glu Ile Asn Cys Thr Arg Pro
      290                 295                 300

  Asn Asn Asn Thr Arg Lys Ser Ile His Ile Gly Pro Gly Arg Ala Phe
  305                 310                 315                     320

  Tyr Thr Ala Gly Asp Ile Ile Gly Asp Ile Arg Gln Ala His Cys Asn
              325                 330                     335

  Ile Ser Arg Ala Asn Trp Asn Asn Thr Leu Arg Gln Ile Val Glu Lys
              340                 345                 350

  Leu Gly Lys Gln Phe Gly Asn Asn Lys Thr Ile Val Phe Asn His Ser
      355                 360                 365

  Ser Gly Gly Asp Pro Glu Ile Val Met His Ser Phe Asn Cys Gly Gly
      370                 375                 380

  Glu Phe Phe Tyr Cys Asn Ser Thr Lys Leu Phe Asn Ser Thr Trp Thr
  385             390                 395                     400

  Trp Asn Asn Ser Thr Trp Asn Asn Thr Lys Arg Ser Asn Asp Thr Glu
              405                 410                     415

  Glu His Ile Thr Leu Pro Cys Arg Ile Lys Gln Ile Ile Asn Met Trp
              420                 425                 430

  Gln Glu Val Gly Lys Ala Met Tyr Ala Pro Pro Ile Arg Gly Gln Ile
      435                 440                 445

  Arg Cys Ser Ser Asn Ile Thr Gly Leu Leu Leu Thr Arg Asp Gly Gly
      450                 455                 460

  Asn Asp Thr Ser Gly Thr Glu Ile Phe Arg Pro Gly Gly Gly Asp Met
  465                 470                 475                     480

  Arg Asp Asn Trp Arg Ser Glu Leu Tyr Lys Tyr Lys Val Val Lys Ile
              485                 490                     495

  Glu Pro Leu Gly Val Ala Pro Thr Lys Ala Lys Arg Arg Val Val Gln
              500                 505                     510

  Ser Glu Lys Ser Ala Val Gly Ile Gly Ala Val Phe Leu Gly Phe Leu
      515                 520                 525

  Gly Ala Ala Gly Ser Thr Met Gly Ala Ala Ser Met Thr Leu Thr Val
</pre>

33

```
                    530                          535                            540

        Gln Ala Arg Leu Leu Leu Ser Gly Ile Val Gln Gln Gln Asn Asn Leu
        545                 550                 555                 560

        Leu Arg Ala Ile Glu Ala Gln Gln His Leu Leu Gln Leu Thr Val Trp
                        565                 570                 575

        Gly Ile Lys Gln Leu Gln Ala Arg Val Leu Ala Val Glu Arg Tyr Leu
                    580                 585                 590

        Lys Asp Gln Gln Leu Leu Gly Ile Trp Gly Cys Ser Gly Lys Leu Ile
                    595                 600                 605

        Cys Thr Thr Thr Val Pro Trp Asn Ala Ser Trp Ser Asn Lys Ser Leu
            610                 615                 620

        Asp Lys Ile Trp Asn Asn Met Thr Trp Met Glu Trp Glu Arg Glu Ile
        625                 630                 635                 640

        Asn Asn Tyr Thr Ser Leu Ile Tyr Thr Leu Ile Glu Glu Ser Gln Asn
                        645                 650                 655

        Gln Gln Glu Lys Asn Glu Gln Glu Leu Leu Glu Leu Asp Lys Trp Ala
                    660                 665                 670

        Ser Leu Trp Asn Trp Phe Asp Ile Ser Asn Trp Leu Trp
                    675                 680                 685
```

<210> 2
<211> 1350
<212> PRT
<213> Artificial Sequence

<220>
<223> Mos1GagPol protein encoded by HIV vaccine drug product Ad26.HIV DP

<400> 2

Met Gly Ala Arg Ala Ser Val Leu Ser Gly Gly Glu Leu Asp Arg Trp
1                   5                   10                  15

Glu Lys Ile Arg Leu Arg Pro Gly Gly Lys Lys Lys Tyr Arg Leu Lys
                20                  25                  30

His Ile Val Trp Ala Ser Arg Glu Leu Glu Arg Phe Ala Val Asn Pro
            35                  40                  45

Gly Leu Leu Glu Thr Ser Glu Gly Cys Arg Gln Ile Leu Gly Gln Leu
        50                  55                  60

Gln Pro Ser Leu Gln Thr Gly Ser Glu Glu Leu Arg Ser Leu Tyr Asn
65                  70                  75                  80

Thr Val Ala Thr Leu Tyr Cys Val His Gln Arg Ile Glu Ile Lys Asp
                85                  90                  95

Thr Lys Glu Ala Leu Glu Lys Ile Glu Glu Glu Gln Asn Lys Ser Lys
                100                 105                 110

Lys Lys Ala Gln Gln Ala Ala Ala Asp Thr Gly Asn Ser Ser Gln Val
                115                 120                 125

Ser Gln Asn Tyr Pro Ile Val Gln Asn Ile Gln Gly Gln Met Val His

                    130                        135                        140

          Gln Ala Ile Ser Pro Arg Thr Leu Asn Ala Trp Val Lys Val Val Glu
          145                 150                 155                 160

          Glu Lys Ala Phe Ser Pro Glu Val Ile Pro Met Phe Ser Ala Leu Ser
                          165                 170                 175

          Glu Gly Ala Thr Pro Gln Asp Leu Asn Thr Met Leu Asn Thr Val Gly
                      180                 185                 190

          Gly His Gln Ala Ala Met Gln Met Leu Lys Glu Thr Ile Asn Glu Glu
                  195                 200                 205

          Ala Ala Glu Trp Asp Arg Val His Pro Val His Ala Gly Pro Ile Ala
          210                 215                 220

          Pro Gly Gln Met Arg Glu Pro Arg Gly Ser Asp Ile Ala Gly Thr Thr
          225                 230                 235                 240

          Ser Thr Leu Gln Glu Gln Ile Gly Trp Met Thr Asn Asn Pro Pro Ile
                          245                 250                 255

          Pro Val Gly Glu Ile Tyr Lys Arg Trp Ile Ile Leu Gly Leu Asn Lys
                      260                 265                 270

          Ile Val Arg Met Tyr Ser Pro Val Ser Ile Leu Asp Ile Arg Gln Gly
                  275                 280                 285

          Pro Lys Glu Pro Phe Arg Asp Tyr Val Asp Arg Phe Tyr Lys Thr Leu
                  290                 295                 300

          Arg Ala Glu Gln Ala Ser Gln Asp Val Lys Asn Trp Met Thr Glu Thr
          305                 310                 315                 320

          Leu Leu Val Gln Asn Ala Asn Pro Asp Cys Lys Thr Ile Leu Lys Ala
                          325                 330                 335

          Leu Gly Pro Ala Ala Thr Leu Glu Glu Met Met Thr Ala Cys Gln Gly
                      340                 345                 350

          Val Gly Gly Pro Gly His Lys Ala Arg Val Leu Ala Glu Ala Met Ser
                      355                 360                 365

          Gln Val Thr Asn Ser Ala Thr Ile Met Met Gln Arg Gly Asn Phe Arg
              370                 375                 380

          Asn Gln Arg Lys Thr Val Lys Cys Phe Asn Cys Gly Lys Glu Gly His
          385                 390                 395                 400

          Ile Ala Lys Asn Cys Arg Ala Pro Arg Lys Lys Gly Cys Trp Lys Cys
                          405                 410                 415

          Gly Lys Glu Gly His Gln Met Lys Asp Cys Thr Glu Arg Gln Ala Asn
                      420                 425                 430

          Phe Leu Gly Lys Ile Trp Pro Ser Asn Lys Gly Arg Pro Gly Asn Phe
                  435                 440                 445

          Leu Gln Asn Arg Pro Glu Pro Thr Ala Pro Pro Glu Glu Ser Phe Arg
              450                 455                 460

          Phe Gly Glu Glu Thr Thr Thr Pro Ser Gln Lys Gln Glu Pro Ile Asp

|     |     |     |     | 465 |     |     |     |     | 470 |     |     |     |     | 475 |     |     |     |     | 480 |

```
            465                   470                   475                   480

Lys Glu Met Tyr Pro Leu Ala Ser Leu Lys Ser Leu Phe Gly Asn Asp
            485                   490                   495

Pro Ser Ser Gln Met Ala Pro Ile Ser Pro Ile Glu Thr Val Pro Val
            500                   505                   510

Lys Leu Lys Pro Gly Met Asp Gly Pro Arg Val Lys Gln Trp Pro Leu
            515                   520                   525

Thr Glu Glu Lys Ile Lys Ala Leu Thr Ala Ile Cys Glu Glu Met Glu
            530                   535                   540

Lys Glu Gly Lys Ile Thr Lys Ile Gly Pro Glu Asn Pro Tyr Asn Thr
545                   550                   555                   560

Pro Val Phe Ala Ile Lys Lys Lys Asp Ser Thr Lys Trp Arg Lys Leu
            565                   570                   575

Val Asp Phe Arg Glu Leu Asn Lys Arg Thr Gln Asp Phe Trp Glu Val
            580                   585                   590

Gln Leu Gly Ile Pro His Pro Ala Gly Leu Lys Lys Lys Lys Ser Val
            595                   600                   605

Thr Val Leu Ala Val Gly Asp Ala Tyr Phe Ser Val Pro Leu Asp Glu
            610                   615                   620

Gly Phe Arg Lys Tyr Thr Ala Phe Thr Ile Pro Ser Thr Asn Asn Glu
625                   630                   635                   640

Thr Pro Gly Ile Arg Tyr Gln Tyr Asn Val Leu Pro Gln Gly Trp Lys
            645                   650                   655

Gly Ser Pro Ala Ile Phe Gln Cys Ser Met Thr Arg Ile Leu Glu Pro
            660                   665                   670

Phe Arg Ala Lys Asn Pro Glu Ile Val Ile Tyr Gln Tyr Met Ala Ala
            675                   680                   685

Leu Tyr Val Gly Ser Asp Leu Glu Ile Gly Gln His Arg Ala Lys Ile
            690                   695                   700

Glu Glu Leu Arg Glu His Leu Leu Lys Trp Gly Phe Thr Thr Pro Asp
705                   710                   715                   720

Lys Lys His Gln Lys Glu Pro Pro Phe Leu Trp Met Gly Tyr Glu Leu
            725                   730                   735

His Pro Asp Lys Trp Thr Val Gln Pro Ile Gln Leu Pro Glu Lys Asp
            740                   745                   750

Ser Trp Thr Val Asn Asp Ile Gln Lys Leu Val Gly Lys Leu Asn Trp
            755                   760                   765

Ala Ser Gln Ile Tyr Pro Gly Ile Lys Val Arg Gln Leu Cys Lys Leu
            770                   775                   780

Leu Arg Gly Ala Lys Ala Leu Thr Asp Ile Val Pro Leu Thr Glu Glu
785                   790                   795                   800

Ala Glu Leu Glu Leu Ala Glu Asn Arg Glu Ile Leu Lys Glu Pro Val
```

```
                    805                     810                     815

        His Gly Val Tyr Tyr Asp Pro Ser Lys Asp Leu Ile Ala Glu Ile Gln
                820                     825                     830

        Lys Gln Gly His Asp Gln Trp Thr Tyr Gln Ile Tyr Gln Glu Pro Phe
                835                     840                     845

        Lys Asn Leu Lys Thr Gly Lys Tyr Ala Lys Met Arg Thr Ala His Thr
                850                     855                     860

        Asn Asp Val Lys Gln Leu Thr Glu Ala Val Gln Lys Ile Ala Met Glu
        865                     870                     875                     880

        Ser Ile Val Ile Trp Gly Lys Thr Pro Lys Phe Arg Leu Pro Ile Gln
                885                     890                     895

        Lys Glu Thr Trp Glu Thr Trp Trp Thr Asp Tyr Trp Gln Ala Thr Trp
                900                     905                     910

        Ile Pro Glu Trp Glu Phe Val Asn Thr Pro Pro Leu Val Lys Leu Trp
                915                     920                     925

        Tyr Gln Leu Glu Lys Asp Pro Ile Ala Gly Val Glu Thr Phe Tyr Val
                930                     935                     940

        Ala Gly Ala Ala Asn Arg Glu Thr Lys Leu Gly Lys Ala Gly Tyr Val
        945                     950                     955                     960

        Thr Asp Arg Gly Arg Gln Lys Ile Val Ser Leu Thr Glu Thr Thr Asn
                965                     970                     975

        Gln Lys Thr Ala Leu Gln Ala Ile Tyr Leu Ala Leu Gln Asp Ser Gly
                980                     985                     990

        Ser Glu Val Asn Ile Val Thr Ala Ser Gln Tyr Ala Leu Gly Ile Ile
                995                     1000                    1005

        Gln Ala Gln Pro Asp Lys Ser Glu Ser Glu Leu Val Asn Gln Ile Ile
                1010                    1015                    1020

        Glu Gln Leu Ile Lys Lys Glu Arg Val Tyr Leu Ser Trp Val Pro Ala
        1025                    1030                    1035                    1040

        His Lys Gly Ile Gly Gly Asn Glu Gln Val Asp Lys Leu Val Ser Ser
                1045                    1050                    1055

        Gly Ile Arg Lys Val Leu Phe Leu Asp Gly Ile Asp Lys Ala Gln Glu
                1060                    1065                    1070

        Glu His Glu Lys Tyr His Ser Asn Trp Arg Ala Met Ala Ser Asp Phe
                1075                    1080                    1085

        Asn Leu Pro Pro Val Val Ala Lys Glu Ile Val Ala Ser Cys Asp Gln
                1090                    1095                    1100

        Cys Gln Leu Lys Gly Glu Ala Met His Gly Gln Val Asp Cys Ser Pro
        1105                    1110                    1115                    1120

        Gly Ile Trp Gln Leu Ala Cys Thr His Leu Glu Gly Lys Ile Ile Leu
                1125                    1130                    1135

        Val Ala Val His Val Ala Ser Gly Tyr Ile Glu Ala Glu Val Ile Pro
```

38

                    1140                    1145                    1150

        Ala Glu Thr Gly Gln Glu Thr Ala Tyr Phe Ile Leu Lys Leu Ala Gly
                1155                1160                1165

        Arg Trp Pro Val Lys Val Ile His Thr Ala Asn Gly Ser Asn Phe Thr
                1170                1175                1180

        Ser Ala Ala Val Lys Ala Ala Cys Trp Trp Ala Gly Ile Gln Gln Glu
        1185                1190                1195                1200

        Phe Gly Ile Pro Tyr Asn Pro Gln Ser Gln Gly Val Val Ala Ser Met
                    1205                1210                1215

        Asn Lys Glu Leu Lys Lys Ile Ile Gly Gln Val Arg Asp Gln Ala Glu
                1220                1225                1230

        His Leu Lys Thr Ala Val Gln Met Ala Val Phe Ile His Asn Phe Lys
                1235                1240                1245

        Arg Lys Gly Gly Ile Gly Gly Tyr Ser Ala Gly Glu Arg Ile Ile Asp
                1250                1255                1260

        Ile Ile Ala Thr Asp Ile Gln Thr Lys Glu Leu Gln Lys Gln Ile Ile
        1265                1270                1275                1280

        Lys Ile Gln Asn Phe Arg Val Tyr Tyr Arg Asp Ser Arg Asp Pro Ile
                    1285                1290                1295

        Trp Lys Gly Pro Ala Lys Leu Leu Trp Lys Gly Glu Gly Ala Val Val
                    1300                1305                1310

        Ile Gln Asp Asn Ser Asp Ile Lys Val Val Pro Arg Arg Lys Val Lys
                1315                1320                1325

        Ile Ile Lys Asp Tyr Gly Lys Gln Met Ala Gly Ala Asp Cys Val Ala
                1330                1335                1340

        Gly Arg Gln Asp Glu Asp
        1345                1350

<210> 3
<211> 1341
<212> PRT
<213> Artificial Sequence

<220>
<223> Mos2GagPol protein encoded by HIV vaccine drug product Ad26.HIV DP

<400> 3

```
Met Gly Ala Arg Ala Ser Ile Leu Arg Gly Gly Lys Leu Asp Lys Trp
1               5                   10                  15

Glu Lys Ile Arg Leu Arg Pro Gly Gly Lys Lys His Tyr Met Leu Lys
            20                  25                  30

His Leu Val Trp Ala Ser Arg Glu Leu Glu Arg Phe Ala Leu Asn Pro
            35                  40                  45

Gly Leu Leu Glu Thr Ser Glu Gly Cys Lys Gln Ile Ile Lys Gln Leu
    50                  55                  60

Gln Pro Ala Leu Gln Thr Gly Thr Glu Glu Leu Arg Ser Leu Phe Asn
```

```
        65                      70                      75                      80
```

Thr Val Ala Thr Leu Tyr Cys Val His Ala Glu Ile Glu Val Arg Asp
                85                      90                      95

Thr Lys Glu Ala Leu Asp Lys Ile Glu Glu Glu Gln Asn Lys Ser Gln
               100                     105                     110

Gln Lys Thr Gln Gln Ala Lys Glu Ala Asp Gly Lys Val Ser Gln Asn
               115                     120                     125

Tyr Pro Ile Val Gln Asn Leu Gln Gly Gln Met Val His Gln Pro Ile
       130                     135                     140

Ser Pro Arg Thr Leu Asn Ala Trp Val Lys Val Ile Glu Glu Lys Ala
145                     150                     155                     160

Phe Ser Pro Glu Val Ile Pro Met Phe Thr Ala Leu Ser Glu Gly Ala
               165                     170                     175

Thr Pro Gln Asp Leu Asn Thr Met Leu Asn Thr Val Gly Gly His Gln
               180                     185                     190

Ala Ala Met Gln Met Leu Lys Asp Thr Ile Asn Glu Glu Ala Ala Glu
       195                     200                     205

Trp Asp Arg Leu His Pro Val His Ala Gly Pro Val Ala Pro Gly Gln
       210                     215                     220

Met Arg Glu Pro Arg Gly Ser Asp Ile Ala Gly Thr Thr Ser Asn Leu
225                     230                     235                     240

Gln Glu Gln Ile Ala Trp Met Thr Ser Asn Pro Pro Ile Pro Val Gly
               245                     250                     255

Asp Ile Tyr Lys Arg Trp Ile Ile Leu Gly Leu Asn Lys Ile Val Arg
               260                     265                     270

Met Tyr Ser Pro Thr Ser Ile Leu Asp Ile Lys Gln Gly Pro Lys Glu
       275                     280                     285

Pro Phe Arg Asp Tyr Val Asp Arg Phe Phe Lys Thr Leu Arg Ala Glu
       290                     295                     300

Gln Ala Thr Gln Asp Val Lys Asn Trp Met Thr Asp Thr Leu Leu Val
305                     310                     315                     320

Gln Asn Ala Asn Pro Asp Cys Lys Thr Ile Leu Arg Ala Leu Gly Pro
               325                     330                     335

Gly Ala Thr Leu Glu Glu Met Met Thr Ala Cys Gln Gly Val Gly Gly
       340                     345                     350

Pro Ser His Lys Ala Arg Val Leu Ala Glu Ala Met Ser Gln Thr Asn
       355                     360                     365

Ser Thr Ile Leu Met Gln Arg Ser Asn Phe Lys Gly Ser Lys Arg Ile
       370                     375                     380

Val Lys Cys Phe Asn Cys Gly Lys Glu Gly His Ile Ala Arg Asn Cys
385                     390                     395                     400

Arg Ala Pro Arg Lys Lys Gly Cys Trp Lys Cys Gly Lys Glu Gly His

```
                        405                      410                      415

        Gln Met Lys Asp Cys Thr Glu Arg Gln Ala Asn Phe Leu Gly Lys Ile
                    420                  425                  430

        Trp Pro Ser His Lys Gly Arg Pro Gly Asn Phe Leu Gln Ser Arg Pro
                    435                  440                  445

        Glu Pro Thr Ala Pro Pro Ala Glu Ser Phe Arg Phe Glu Glu Thr Thr
                    450                  455                  460

        Pro Ala Pro Lys Gln Glu Pro Lys Asp Arg Glu Pro Leu Thr Ser Leu
        465                  470                  475                  480

        Arg Ser Leu Phe Gly Ser Asp Pro Leu Ser Gln Met Ala Pro Ile Ser
                    485                  490                  495

        Pro Ile Glu Thr Val Pro Val Lys Leu Lys Pro Gly Met Asp Gly Pro
                    500                  505                  510

        Lys Val Lys Gln Trp Pro Leu Thr Glu Glu Lys Ile Lys Ala Leu Val
                    515                  520                  525

        Glu Ile Cys Thr Glu Met Glu Lys Glu Gly Lys Ile Ser Lys Ile Gly
                    530                  535                  540

        Pro Glu Asn Pro Tyr Asn Thr Pro Ile Phe Ala Ile Lys Lys Lys Asp
        545                  550                  555                  560

        Ser Thr Lys Trp Arg Lys Leu Val Asp Phe Arg Glu Leu Asn Lys Arg
                    565                  570                  575

        Thr Gln Asp Phe Trp Glu Val Gln Leu Gly Ile Pro His Pro Ala Gly
                    580                  585                  590

        Leu Lys Lys Lys Lys Ser Val Thr Val Leu Ala Val Gly Asp Ala Tyr
                    595                  600                  605

        Phe Ser Val Pro Leu Asp Glu Asp Phe Arg Lys Tyr Thr Ala Phe Thr
            610                  615                  620

        Ile Pro Ser Ile Asn Asn Glu Thr Pro Gly Ile Arg Tyr Gln Tyr Asn
        625                  630                  635                  640

        Val Leu Pro Gln Gly Trp Lys Gly Ser Pro Ala Ile Phe Gln Ser Ser
                    645                  650                  655

        Met Thr Lys Ile Leu Glu Pro Phe Arg Lys Gln Asn Pro Asp Ile Val
                    660                  665                  670

        Ile Tyr Gln Tyr Met Ala Ala Leu Tyr Val Gly Ser Asp Leu Glu Ile
                    675                  680                  685

        Gly Gln His Arg Thr Lys Ile Glu Glu Leu Arg Gln His Leu Leu Arg
                    690                  695                  700

        Trp Gly Phe Thr Thr Pro Asp Lys Lys His Gln Lys Glu Pro Pro Phe
        705                  710                  715                  720

        Leu Trp Met Gly Tyr Glu Leu His Pro Asp Lys Trp Thr Val Gln Pro
                    725                  730                  735

        Ile Val Leu Pro Glu Lys Asp Ser Trp Thr Val Asn Asp Ile Gln Lys
```

740                          745                          750

Leu Val Gly Lys Leu Asn Trp Ala Ser Gln Ile Tyr Ala Gly Ile Lys
        755                 760                 765

Val Lys Gln Leu Cys Lys Leu Leu Arg Gly Thr Lys Ala Leu Thr Glu
        770                 775                 780

Val Val Pro Leu Thr Glu Glu Ala Glu Leu Glu Leu Ala Glu Asn Arg
785                 790                 795                 800

Glu Ile Leu Lys Glu Pro Val His Gly Val Tyr Tyr Asp Pro Ser Lys
                805                 810                 815

Asp Leu Ile Ala Glu Ile Gln Lys Gln Gly Gln Gly Gln Trp Thr Tyr
                820                 825                 830

Gln Ile Tyr Gln Glu Pro Phe Lys Asn Leu Lys Thr Gly Lys Tyr Ala
        835                 840                 845

Arg Met Arg Gly Ala His Thr Asn Asp Val Lys Gln Leu Thr Glu Ala
        850                 855                 860

Val Gln Lys Ile Ala Thr Glu Ser Ile Val Ile Trp Gly Lys Thr Pro
865                 870                 875                 880

Lys Phe Lys Leu Pro Ile Gln Lys Glu Thr Trp Glu Ala Trp Trp Thr
                885                 890                 895

Glu Tyr Trp Gln Ala Thr Trp Ile Pro Glu Trp Glu Phe Val Asn Thr
                900                 905                 910

Pro Pro Leu Val Lys Leu Trp Tyr Gln Leu Glu Lys Glu Pro Ile Val
                915                 920                 925

Gly Ala Glu Thr Phe Tyr Val Ala Gly Ala Ala Asn Arg Glu Thr Lys
        930                 935                 940

Leu Gly Lys Ala Gly Tyr Val Thr Asp Arg Gly Arg Gln Lys Val Val
945                 950                 955                 960

Ser Leu Thr Asp Thr Thr Asn Gln Lys Thr Ala Leu Gln Ala Ile His
                965                 970                 975

Leu Ala Leu Gln Asp Ser Gly Leu Glu Val Asn Ile Val Thr Ala Ser
        980                 985                 990

Gln Tyr Ala Leu Gly Ile Ile Gln Ala Gln Pro Asp Lys Ser Glu Ser
        995                 1000                1005

Glu Leu Val Ser Gln Ile Ile Glu Gln Leu Ile Lys Lys Glu Lys Val
        1010                1015                1020

Tyr Leu Ala Trp Val Pro Ala His Lys Gly Ile Gly Gly Asn Glu Gln
1025                1030                1035                1040

Val Asp Lys Leu Val Ser Arg Gly Ile Arg Lys Val Leu Phe Leu Asp
                1045                1050                1055

Gly Ile Asp Lys Ala Gln Glu Glu His Glu Lys Tyr His Ser Asn Trp
                1060                1065                1070

Arg Ala Met Ala Ser Glu Phe Asn Leu Pro Pro Ile Val Ala Lys Glu

```
                    1075                    1080                    1085

        Ile Val Ala Ser Cys Asp Lys Cys Gln Leu Lys Gly Glu Ala Ile His
            1090                1095                1100

        Gly Gln Val Asp Cys Ser Pro Gly Ile Trp Gln Leu Ala Cys Thr His
        1105                1110                1115                1120

        Leu Glu Gly Lys Val Ile Leu Val Ala Val His Val Ala Ser Gly Tyr
                        1125                1130                1135

        Ile Glu Ala Glu Val Ile Pro Ala Glu Thr Gly Gln Glu Thr Ala Tyr
                    1140                1145                1150

        Phe Leu Leu Lys Leu Ala Gly Arg Trp Pro Val Lys Thr Ile His Thr
                1155                1160                1165

        Ala Asn Gly Ser Asn Phe Thr Ser Ala Thr Val Lys Ala Ala Cys Trp
                1170                1175                1180

        Trp Ala Gly Ile Lys Gln Glu Phe Gly Ile Pro Tyr Asn Pro Gln Ser
        1185                1190                1195                1200

        Gln Gly Val Val Ala Ser Ile Asn Lys Glu Leu Lys Lys Ile Ile Gly
                        1205                1210                1215

        Gln Val Arg Asp Gln Ala Glu His Leu Lys Thr Ala Val Gln Met Ala
                    1220                1225                1230

        Val Phe Ile His Asn Phe Lys Arg Lys Gly Gly Ile Gly Glu Tyr Ser
                    1235                1240                1245

        Ala Gly Glu Arg Ile Val Asp Ile Ile Ala Ser Asp Ile Gln Thr Lys
                1250                1255                1260

        Glu Leu Gln Lys Gln Ile Thr Lys Ile Gln Asn Phe Arg Val Tyr Tyr
        1265                1270                1275                1280

        Arg Asp Ser Arg Asp Pro Leu Trp Lys Gly Pro Ala Lys Leu Leu Trp
                    1285                1290                1295

        Lys Gly Glu Gly Ala Val Val Ile Gln Asp Asn Ser Asp Ile Lys Val
                    1300                1305                1310

        Val Pro Arg Arg Lys Ala Lys Ile Ile Arg Asp Tyr Gly Lys Gln Met
                1315                1320                1325

        Ala Gly Asp Asp Cys Val Ala Ser Arg Gln Asp Glu Asp
            1330                1335                1340
```

<210> 4
<211> 17
<212> PRT
<213> Artificial Sequence

<220>
<223> Proteotypic peptide Env4

<400> 4

```
        Ala Ile Glu Ala Gln Gln His Leu Leu Gln Leu Thr Val Trp Gly Ile
        1               5                   10                  15

        Lys
```

<210> 5
<211> 15
<212> PRT
<213> Artificial Sequence

<220>
<223> Proteotypic peptide Mosl.3

<400> 5

```
        Ile Gly Pro Glu Asn Pro Tyr Asn Thr Pro Val Phe Ala Ile Lys
        1               5                   10                  15
```

<210> 6
<211> 15
<212> PRT
<213> Artificial Sequence

<220>
<223> Proteotypic peptide Mos2.1

<400> 6

```
        Ile Gly Pro Glu Asn Pro Tyr Asn Thr Pro Ile Phe Ala Ile Lys
        1               5                   10                  15
```

<210> 7
<211> 17
<212> PRT
<213> Artificial Sequence

<220>
<223> Proteotypic peptide Mos2.2

<400> 7

```
        Tyr Thr Ala Phe Thr Ile Pro Ser Ile Asn Asn Glu Thr Pro Gly Ile
        1               5                   10                  15

        Arg
```

<210> 8
<211> 16
<212> PRT
<213> Artificial Sequence

<220>
<223> Proteotypic peptide Act1

<400> 8

```
        Ser Tyr Glu Leu Pro Asp Gly Gln Val Ile Thr Ile Gly Asn Glu Arg
        1               5                   10                  15
```

<210> 9
<211> 14
<212> PRT
<213> Artificial Sequence

<220>
<223> Proteotypic peptide G3P1

<400> 9

```
Leu Ile Ser Trp Tyr Asp Asn Glu Phe Gly Tyr Ser Asn Arg
        1               5                   10
```

<210> 10
<211> 13
<212> PRT
<213> Artificial Sequence

<220>
<223> Proteotypic peptide G3P3a

<400> 10

```
Ala Val Gly Lys Val Ile Pro Glu Glu Leu Asn Gly Lys
1               5               10
```

<210> 11
<211> 15
<212> PRT
<213> Artificial Sequence

<220>
<223> Proteotypic peptide VII1

<400> 11

```
Thr Thr Val Asp Asp Val Ile Asp Ser Val Val Ala Asp Ala Arg
1               5                   10                  15
```

<210> 12
<211> 13
<212> PRT
<213> Artificial Sequence

<220>
<223> Proteotypic peptide Env1

<400> 12

```
Glu Ala Thr Thr Thr Leu Phe Glx Ala Ser Asp Ala Lys
1               5                   10
```

<210> 13
<211> 20
<212> PRT

<213> Artificial Sequence

<220>
<223> Proteotypic peptide Env2

<400> 13

Val Ser Phe Glu Pro Ile Pro Ile His Tyr Glx Ala Pro Ala Gly Phe
1               5                   10                  15

Ala Ile Leu Lys
            20

<210> 14
<211> 13
<212> PRT
<213> Artificial Sequence

<220>
<223> Proteotypic peptides Env3 and Env3a

<400> 14

Ala Phe Tyr Thr Ala Gly Asp Ile Ile Gly Asp Ile Arg

            1               5                   10

<210> 15
<211> 15
<212> PRT
<213> Artificial Sequence

<220>
<223> Proteotypic peptide Mos1.1

<400> 15

Phe Ala Val Asn Pro Gly Leu Leu Glu Thr Ser Glu Gly Glx Arg
1               5                   10                  15

<210> 16
<211> 15
<212> PRT
<213> Artificial Sequence

<220>
<223> Proteotypic peptide Mosl.2

<400> 16

Ser Leu Tyr Asn Thr Val Ala Thr Leu Tyr Glx Val His Gln Arg
1               5                   10                  15

<210> 17
<211> 11
<212> PRT

<213> Artificial Sequence

<220>
<223> Proteotypic peptide Mosl.4

<400> 17

```
          Ile Val Ser Leu Thr Glu Thr Thr Asn Gln Lys
          1               5                   10
```

<210> 18
<211> 11
<212> PRT
<213> Artificial Sequence

<220>
<223> Proteotypic peptides Mos2.3 and Mos2.3a

<400> 18

```
          Ile Ala Thr Glu Ser Ile Val Ile Trp Gly Lys
          1               5                   10
```

<210> 19
<211> 11
<212> PRT
<213> Artificial Sequence

<220>
<223> Proteotypic peptide Mos2.4

<400> 19

```
          Val Val Ser Leu Thr Asp Thr Thr Asn Gln Lys
          1               5                   10
```

<210> 20
<211> 15
<212> PRT
<213> Artificial Sequence

<220>
<223> Proteotypic peptide Mos2.5

<400> 20

```
        Phe Ala Leu Asn Pro Gly Leu Leu Glu Thr Ser Glu Gly Glx Lys
        1             5                   10                      15
```

<210> 21
<211> 8
<212> PRT
<213> Artificial Sequence

<220>
<223> Proteotypic peptide G3P2

<400> 21

```
                        Val Ile Pro Glu Leu Asn Gly Lys
                        1               5
```

<210> 22
<211> 13
<212> PRT
<213> Artificial Sequence

<220>
<223> Proteotypic peptide G3P3a

<400> 22

```
                Ala Val Gly Lys Val Ile Pro Glu Glu Leu Asn Gly Lys
                1           5               10
```

<210> 23
<211> 11
<212> PRT
<213> Artificial Sequence

<220>
<223> Proteotypic peptide Hex1

<400> 23

```
                    Ala Thr Asp Thr Tyr Phe Ser Leu Gly Asn Lys
                    1           5               10
```

<210> 24
<211> 13
<212> PRT
<213> Artificial Sequence

<220>
<223> Proteotypic peptide IX1

<400> 24

```
                    Leu Leu Ala Leu Leu Ala Glu Leu Glu Ala Leu Ser Arg
                    1           5               10
```

<210> 25
<211> 10
<212> PRT
<213> Artificial Sequence

<220>
<223> MosI.Env proteotypic peptide

<400> 25

```
                Ile Glu Pro Leu Gly Val Ala Pro Thr Lys
                1           5               10
```

<210> 26
<211> 711

<220>
<223> Mos2S.Env

<400> 26

```
Met Arg Val Arg Gly Met Leu Arg Asn Trp Gln Gln Trp Trp Ile Trp
1               5                   10                  15

Ser Ser Leu Gly Phe Trp Met Leu Met Ile Tyr Ser Val Met Gly Asn
            20                  25                  30

Leu Trp Val Thr Val Tyr Tyr Gly Val Pro Val Trp Lys Asp Ala Lys
            35                  40                  45

Thr Thr Leu Phe Cys Ala Ser Asp Ala Lys Ala Tyr Glu Lys Glu Val
        50                  55                  60

His Asn Val Trp Ala Thr His Ala Cys Val Pro Thr Asp Pro Asn Pro
65                  70                  75                  80

Gln Glu Ile Val Leu Gly Asn Val Thr Glu Asn Phe Asn Met Trp Lys
                85                  90                  95

Asn Asp Met Val Asp Gln Met His Glu Asp Ile Ile Ser Leu Trp Asp
            100                 105                 110

Ala Ser Leu Glu Pro Cys Val Lys Leu Thr Pro Leu Cys Val Thr Leu
        115                 120                 125

Asn Cys Arg Asn Val Arg Asn Val Ser Ser Asn Gly Thr Tyr Asn Ile
    130                 135                 140

Ile His Asn Glu Thr Tyr Lys Glu Met Lys Asn Cys Ser Phe Asn Ala
145                 150                 155                 160

Thr Thr Val Val Glu Asp Arg Lys Gln Lys Val His Ala Leu Phe Tyr
                165                 170                 175

Arg Leu Asp Ile Val Pro Leu Asp Glu Asn Asn Ser Ser Glu Lys Ser
            180                 185                 190

Ser Glu Asn Ser Ser Glu Tyr Tyr Arg Leu Ile Asn Cys Asn Thr Ser
            195                 200                 205

Ala Ile Thr Gln Ala Cys Pro Lys Val Ser Phe Asp Pro Ile Pro Ile
    210                 215                 220

His Tyr Cys Ala Pro Ala Gly Tyr Ala Ile Leu Lys Cys Asn Asn Lys
225                 230                 235                 240

Thr Phe Asn Gly Thr Gly Pro Cys Asn Asn Val Ser Thr Val Gln Cys
```

245                    250                    255

Thr His Gly Ile Lys Pro Val Val Ser Thr Gln Leu Leu Leu Asn Gly
        260               265               270

Ser Leu Ala Glu Glu Glu Ile Ile Ile Arg Ser Glu Asn Leu Thr Asn
        275               280               285

Asn Ala Lys Thr Ile Ile Val His Leu Asn Glu Thr Val Asn Ile Thr
        290               295               300

Cys Thr Arg Pro Asn Asn Asn Thr Arg Lys Ser Ile Arg Ile Gly Pro
305                310               315               320

Gly Gln Thr Phe Tyr Ala Thr Gly Asp Ile Ile Gly Asp Ile Arg Gln
        325               330               335

Ala His Cys Asn Leu Ser Arg Asp Gly Trp Asn Lys Thr Leu Gln Gly
        340               345               350

Val Lys Lys Lys Leu Ala Glu His Phe Pro Asn Lys Thr Ile Lys Phe
        355               360               365

Ala Pro His Ser Gly Gly Asp Leu Glu Ile Thr Thr His Thr Phe Asn
370                375               380

Cys Arg Gly Glu Phe Phe Tyr Cys Asn Thr Ser Asn Leu Phe Asn Glu
385                390               395               400

Ser Asn Ile Glu Arg Asn Asp Ser Ile Ile Thr Leu Pro Cys Arg Ile
        405               410               415

Lys Gln Ile Ile Asn Met Trp Gln Glu Val Gly Arg Ala Ile Tyr Ala
        420               425               430

Pro Pro Ile Ala Gly Asn Ile Thr Cys Arg Ser Asn Ile Thr Gly Leu
        435               440               445

Leu Leu Thr Arg Asp Gly Gly Ser Asn Asn Gly Val Pro Asn Asp Thr
        450               455               460

Glu Thr Phe Arg Pro Gly Gly Gly Asp Met Arg Asn Asn Trp Arg Ser
465                470               475               480

Glu Leu Tyr Lys Tyr Lys Val Val Glu Val Lys Pro Leu Gly Val Ala
        485               490               495

Pro Thr Glu Ala Lys Arg Arg Val Val Glu Arg Glu Lys Arg Ala Val
        500               505               510

Gly Ile Gly Ala Val Phe Leu Gly Ile Leu Gly Ala Ala Gly Ser Thr
        515               520               525

Met Gly Ala Ala Ser Ile Thr Leu Thr Val Gln Ala Arg Gln Leu Leu
        530               535               540

Ser Gly Ile Val Gln Gln Gln Ser Asn Leu Leu Arg Ala Ile Glu Ala
545                550               555               560

Gln Gln His Met Leu Gln Leu Thr Val Trp Gly Ile Lys Gln Leu Gln
        565               570               575

Thr Arg Val Leu Ala Ile Glu Arg Tyr Leu Gln Asp Gln Gln Leu Leu

```
                580                      585                        590

        Gly Leu Trp Gly Cys Ser Gly Lys Leu Ile Cys Thr Thr Ala Val Pro
                595                 600             605

        Trp Asn Thr Ser Trp Ser Asn Lys Ser Gln Thr Asp Ile Trp Asp Asn
                610                 615             620

        Met Thr Trp Met Gln Trp Asp Lys Glu Ile Gly Asn Tyr Thr Gly Glu
        625             630             635                 640

        Ile Tyr Arg Leu Leu Glu Glu Ser Gln Asn Gln Gln Glu Lys Asn Glu
                645                 650             655

        Lys Asp Leu Leu Ala Leu Asp Ser Trp Asn Asn Leu Trp Asn Trp Phe
                660                 665             670

        Ser Ile Ser Lys Trp Leu Trp Tyr Ile Lys Ile Phe Ile Met Ile Val
                675                 680             685

        Gly Gly Leu Ile Gly Leu Arg Ile Ile Phe Ala Val Leu Ser Ile Val
                690                 695                 700

        Asn Arg Val Arg Gln Gly Tyr
        705                 710
```

<210> 27
<211> 10
<212> PRT
<213> Artificial Sequence

<220>
<223> Mos2S.Env proteotypic peptide

<220>
<221> Site
<222> 5
<223> Xaa is carbamidomethylated cysteine

<400> 27

```
        Thr Thr Leu Phe Xaa Ala Ser Asp Ala Lys
        1               5               10
```

**Description**

**TECHNICAL FIELD**

The invention is in the field of assays for determining the protein expression levels of recombinant expression systems.

**BACKGROUND**

Mass spectrometry is a technique that has been used for the quantification of e.g. hemagglutinin (HA) and neuraminidase (NA) in influenza virus preparations [77, 78, 79]. The proteins that are measured are either located on an influenza virus or are originating from purified virus preparations (abstract, [77]). The virus preparations can be either vaccine compositions comprising purified influenza HA or NA proteins (p.522, right column, first paragraph, (781), purified virus (p.523, left column, first paragraph, (78]), inactivated whole virus preparations, or virus samples in allantoic fluid (p.4090, left column, materials and methods section, first paragraph, [79]). The mass spectrometry is typically not performed on samples taken directly from a cell culture that was infected by a viral vector or a nucleic acid vaccine, and wherein the proteins are expressed from said viral vector or nucleic acid. Similarly, methods for determining the level of recombinant proteins using mass spectrometry have been disclosed, wherein the recombinant proteins are present in a purified form (see e.g. paragraphs [047] or [0049] in [80]. The use of mass spectrometry for determining the level of recombinant proteins from a proteasome digest of synthesized, purified peptides, has been disclosed as well (paragraph [0020] in [81]).

Recombinant expression is a fundamental technique in molecular biology. It is the expression of a protein from a gene which is in a context different from that in which it naturally occurs. The resultant protein is generally referred to as a recombinant protein.

The presence or absence of the expression of a recombinant protein can be assessed by looking for bands on an SDS-PAGE gel of a sample from the expression system expressing the protein. A quantitative measure of the level of recombinant expression is obtained by using an enzyme-linked immunosorbent assays (ELISA) [1,2]. An ELISA relies heavily on the successful generation of selective and specific antibodies (typically monoclonal antibodies) against the proteins being expressed in the system. However, the generation of these antibodies is often cumbersome and labour and cost-intensive, and suitably specific antibodies cannot always be generated.

Alternatives to ELISA for quantitative analysis include flow cytometry (e.g. fluorescence-activated cell sorting (FACS))[3], but this technique also requires the use of antibodies.

The reliance on antibodies in methods of quantifying proteins suffers from shortfalls where the sample being quantified contains proteins with high sequence identity. Here, many antibodies cannot distinguish between the closely related proteins, and in some instances it may not even be possible to generate antibodies with sufficient specificity and affinity against an antigen so that the antibody is an effective reagent in these antibody-dependent assays. One particular situation where multiple highly related proteins can be expressed together in a recombinant expression system is during the potency testing of nucleic acid vaccine compositions and vector vaccine compositions.

There therefore remains a need for further and improved alternatives for evaluating the level of recombinant expression in recombinant expression systems.

**DESCRIPTION OF THE INVENTION**

Contrary to established antibody-reliant methods for quantifying proteins, the inventors have developed a mass spectrometry-based approach to quantify the expression of recombinant proteins in expression systems, such as cell cultures used during potency testing of nucleic acid vaccine compositions and vector vaccine compositions. Accordingly, the invention provides a method for determining the level of protein expression by a recombinant expression system, comprising the step of quantifying a recombinant protein expressed by the system using mass spectrometry.

Vaccination is no longer exclusively focussed on the direct delivery of antigens to a subject in order to induce an immune response. First generation vaccines relied on weakened or killed whole organisms. The immune system directly recognised the administered organism, and an immune response is raised against it. Second generation vaccines are based on defined protein or carbohydrate components. As for first generation vaccines, the immune system directly recognises the administered components, and an immune response is raised.

The third generation of vaccines are based on introducing genetically engineered nucleic acids encoding proteins into a subject (both DNA and RNA vaccines have been generated [4]). When the nucleic acid is introduced into a subject, the protein synthesis machinery in the subject's cells recognises one or more promoters in the nucleic acid and then begins to produce the proteins encoded by the nucleic acid *in situ* in the subject. The expressed protein is then processed by cells in which it is expressed, and is presented on the cell surface, whereupon it can be detected by immune cells in the subject, and thereby induces an immune response. Accordingly, here, it is not the composition that is administered to the subject that directly raises an immune response, but instead the protein encoded by the nucleic acid that is administered. A nucleic acid vaccine composition can be made that produces a number of different proteins. This can be achieved by using a single nucleic acid component that encodes a number of proteins. Alternatively, different nucleic acid components each encoding different proteins can be combined to generate the nucleic acid vaccine composition. The proteins encoded by the nucleic acid components of the vaccine are typically antigens from pathogenic organisms, but vaccines can also be used to express tumour antigens, to prompt the subject's immune system to attack malignancies. Nucleic acid vaccines are advantageous in that they can elicit both good B and good T cell responses.

Vector vaccine compositions have been developed to facilitate the introduction of nucleic acids into cells, whereupon the nucleic acids act in the same manner as the nucleic acid vaccines discussed in the previous paragraph. Most commonly, viral vector components are used, which contain genes encoding the proteins for expression in the subject, and often in a replication deficient or attenuated virus. Here, the viral vector vaccine composition is administered to a subject, and the viral vector component infects cells in the subject and thereby introduces the nucleic acid into the cells of the subject.

Nucleic acid vaccination and vector vaccination therefore pose challenges not experienced with first and second generation vaccines, because the immune response is not against the vaccine components themselves, but rather against proteins encoded by the vaccine components as produced *in situ*. It is therefore not possible to test the immunogenicity of these vaccine compositions before the clinical trials. Thus, as a first step in the testing of such vaccine compositions, the regulatory authorities require the determination of how effectively the vaccine composition causes expression of the protein(s) encoded by its component(s) - *i.e.* the vaccine composition's *in vitro* potency. This would indicate that the vaccine composition is able to generate sufficient protein in cells to elicit an immune response should that vaccine be administered to a subject. For viral vector vaccine compositions, their potency also depends on how effectively the viral vector component(s) can infect the cells before expression of the encoded protein(s) even occurs. To determine the effectiveness of expression of the nucleic acid or vector vaccine compositions, the vaccine components are introduced into a recombinant expression system, for example a cell culture, and the expression of proteins encoded by the vaccine components is then determined. Often, the cell culture is composed of cells in which the viral vector vaccine or nucleic acid vaccine cannot replicate (e.g. cells which the viral vector vaccine is only able to infect, not replicate in).

The inventors have discovered that mass spectrometry is particularly advantageous for determining the expression of proteins in a recombinant expression system, such as proteins encoded by the components of nucleic acid or vector vaccine compositions. These advantages arise chiefly because MS is highly sensitive, in that it can be used to detect individually the levels of closely related proteins, and because it is very amenable to screening of multiple proteins in parallel from the same sample. Accordingly, the inventors determined that the MS-based methods are particularly suitable for determining the expression even of closely related proteins from a single nucleic acid vaccine composition or viral vector vaccine compositions. This situation is often encountered in nucleic acid and viral vector vaccines, where a single vaccine may contain genes encoding a number of closely related proteins, *e.g.* the same protein from different strains of an infectious agent, such as different strains of influenza or different HIV strains.

This problem is not faced for second generation (*e.g.* subunit) vaccines, because each of the related proteins combined together in the vaccine can be expressed separately and tested separately (*e.g.* quantified by ELISA), before being combined in the protein-based vaccine composition administered to a subject. In contrast, where each of the closely related proteins is encoded in the same nucleic acid vaccine composition or viral vector vaccine composition then they necessarily are all expressed together in a subject when the vaccine composition is administered to that subject. It is therefore necessary to use a technique for measuring the expression of each of the different proteins when they are present together in a mixture (and therefore mimics the situation that occurs when the vaccine is administered to a subject).

For instance, the inventors found that MS can advantageously quantify two genes that are highly similar that are encoded in the same viral vaccine vector composition (see Example 1 below). The two proteins, Mos1GagPol and Mos2GagPol, which contain a mosaic of epitopes from the Gag and Pol proteins of HIV [5], have 89% sequence identity. The differences in the protein sequences are spread throughout the entire sequence as single amino acid changes. These isolated sequence differences mean that distinctive epitopes are unlikely to be present for raising antibodies that are specific and selective for each protein - if an antibody were employed, the detected result would be dependent on the concentration of both. In the absence of antibodies that can distinguish the two proteins, it would not be possible to quantify each protein individually using conventional methods (and so it would not be possible to perform a full assessment of the potency of each component in the vaccine). Using the method of the invention (of determining the level of protein expression by a recombinant expression system, comprising the step of quantifying a recombinant protein expressed by the system using mass spectrometry) thereby enables the accurate quantitation of such closely related proteins.

The method of the invention may be a multiplex assay that simultaneously quantifies the expression levels of multiple recombinant proteins from a recombinant expression system, *e.g.* as expressed by a nucleic acid vaccine composition or viral vector vaccine composition in a cell culture (either because a component of the composition encodes multiple recombinant proteins, or because the composition comprises multiple different components each encoding different recombinant proteins, such as wherein the viral vector vaccine composition composes multiple different adenovirus vector vaccine components, for example wherein each adenovirus vector vaccine component encodes one transgene). In some embodiments, the cell culture is composed of cells in which the viral vector vaccine or nucleic acid vaccine cannot replicate. The invention therefore also provides a method for quantifying the protein expression of a recombinant expression system expressing multiple recombinant proteins, comprising the step of quantifying recombinant proteins expressed by the system using mass spectrometry. In some embodiments, therefore, the method is used to simultaneously quantify at least 2, at least 3, at least 4, at least 5, at least 6, at least 7, at least 8, at least 9, at least 10, at least 20, at least 50, or at least 100 recombinant proteins. The invention is particularly useful for quantifying a recombinant protein that is highly similar to other proteins in the same sample for the mass spectrometry analysis. The recombinant protein to be quantified may have a sequence identity of x with another protein in the same sample for the mass spectrometry analysis, where x may be $\geq 60\%$, $\geq 70\%$, $\geq 80\%$, $\geq 90\%$, $\geq 95\%$, $\geq 98\%$ or $\geq 99\%$. The other protein may be: (i) a further recombinant protein expressed from the same expression construct (*e.g.* nucleic acid vaccine component, viral vector vaccine component or an expression plasmid), (ii) a protein from the cell in which the recombinant protein is expressed, (iii) a contaminant protein, or (iv) if a second or further expression construct (*e.g.* a further nucleic acid vaccine component, viral vector vaccine component or expression plasmid) is used to encode additional genes encoding recombinant proteins that are expressed in the cell culture, a protein from the second or further expression construct.

MS is also advantageous over conventional methods for assessing nucleic acid or viral vector vaccine components and compositions because it can identify and quantify the protein of interest within the complexity of the cellular protein matrix (containing further recombinant proteins or proteins from the cellular genome, and/or proteins from the cell). Accordingly, in some embodiments, the recombinant expression system is a cell culture and the cell culture is lysed to form a cell lysate that is analysed by mass spectrometry to quantify the recombinant protein, optionally wherein the cell lysate is a clarified cell lysate. When the cell lysate is analysed, this therefore includes many cellular proteins, and the method of the invention is advantageous because it can quantify a recombinant protein when that protein is only one in thousands of proteins in the sample subjected to MS. No additional steps are necessary to purify the recombinant protein from other proteins in the cell lysate. This ability is of particular assistance when a viral vector vaccine or nucleic acid vaccine comprises a tumour antigen that represents a mutated form of the wildtype protein (*e.g.* a mutant form of human protein, such as p53, expressed in a human cell line).

A further advantage of using an immune reagent-free (*e.g.* antibody-free) MS-based approach is that it provides a more robust approach for assessing the expression of recombinant proteins. A monoclonal antibody recognises only a single epitope, so the accuracy of the quantification will depend critically on the specificity and sensitivity of that antibody. In contrast, the MS-based approach in the invention assesses multiple fragments from a protein, so this increases the accuracy of identifying, and hence quantifying, the recombinant protein. Thus, in some embodiments, the method of the invention is immune reagent-free. An immune reagent-free method allows the quantification of multiple genes of interest at the same time, without the extra costs (both money and time) of developing further antibodies to the same antigen as would be necessary in the case of conventional methods.

As will be immediately apparent, although the invention has particular application in assessing expression of proteins encoded by nucleic acid vaccine components and vector vaccine components as discussed above, the technique is applicable to the assessment of any recombinant protein in any recombinant expression system.

### *Potency testing of nucleic acid and vector vaccines*

The method of the invention allows the user to assess of the ability of a nucleic acid vaccine composition, viral vector vaccine composition or the like (*e.g.* bacterial or yeast vector vaccines) to induce protein expression in a cell, and thereby infer how effective that vaccine composition would be if it were administered to a living subject as a pharmaceutical.

Potency testing is particularly important for vector vaccine compositions, because as noted above, the potency of these vector vaccine compositions is composed of two important features: how effectively the vector component transfers the nucleic acid it contains to cells (for a viral vector vaccine, how effectively the viral vector components infect cells), and how well the proteins encoded by the vector components are then expressed. The infectivity of a viral vector component and the effectiveness of expression of the encoded proteins can be affected by the method of producing the viral vector vaccine component, for example the packaging cell line used to produce the viral vector vaccine component or culture conditions in which that packaging cell line is grown when producing the viral vector vaccine component, or specific purification unit operations affecting the vector vaccine component integrity. Also, during long term storage, and at the end of shelf-life, potency testing is required to ensure the quality of the viral vector vaccine component and so batch-to-batch variability in vaccine component production runs may be seen.

Typically, during the manufacture of a vaccine component, the product is produced from multiple production runs in the same or in different production vessels. The methods of the invention therefore find application in analysing samples from different batches. By performing the method of the invention on a recombinant expression system composed of a cell transfected/transformed with a nucleic acid vaccine composition or infected with a viral vector vaccine composition (*e.g.* a particular batch of that vaccine), and by comparing the results with those obtained using a standard or reference batch of the vaccine of known potency, it is possible to determine the relative potency of the test batch of vaccine. This value can be used as a parameter for determining whether a manufactured batch of the vaccine is suitable for release to the public, or whether it has experienced a production failure and so should not be used. Alternatively, an absolute level of protein expression may be set as the criterion for release of the vaccine to the public.

All the following methods referring to "methods of the invention" refer to the methods as claimed, encompassing all claimed method steps. Embodiments are considered as embodiments of the description and not as embodiments of the invention, which is defined by the claims. Accordingly, the invention provides a method of testing the potency of a nucleic acid vaccine composition or viral vector vaccine composition comprising determining the expression of a protein encoded by the vaccine composition using the MS-based method of determining protein expression according to the invention. The invention provides a method of potency testing a nucleic acid vaccine composition comprising: (i) introducing the vaccine composition into a cell culture; and (ii) quantifying the level of expression of a recombinant protein encoded by the vaccine composition by mass spectrometry. The invention provides a method of potency testing a viral vector vaccine composition comprising: (i) infecting a cell culture with the viral vector vaccine composition; and (ii) quantifying the level of expression of a recombinant protein encoded by the vaccine composition by mass spectrometry. The methods described herein can also be used for individually testing the potency of single components of multivalent compositions before they are mixed to form the multivalent composition. In some embodiments, the cell culture is composed of cells in which the viral vector vaccine cannot replicate.

The invention also provides a method of determining the infectivity of a viral vector component by quantifying the intracellular level of a protein from the viral vector component in a recombinant expression system (*e.g.* a cell culture) infected with the viral vector component, such as a replication deficient viral vector component. Here, where the viral vector cannot replicate within the cells then the intracellular level of the proteins of the viral vector component (*e.g.* the capsid proteins that make up the viral particles themselves, not the proteins encoded by the nucleic acid carried in the viral particles) is a measure of the number of viral particles that have infected cells in the recombinant expression system. The intracellular expression level can be determined by preparing a cell pellet, for example by washing the cells (*e.g.* 3 times in ice cold PBS), followed by centrifugation and discarding the supernatant of the cell culture. The method of determining the infectivity of a viral vector component can comprise part of the method of the invention of determining the potency of a viral vector vaccine composition, as discussed in the preceding paragraphs and elsewhere herein. The potency and infectivity can be determined in the same method (*e.g.* by determining intracellular protein level of the viral vector component when the expression of the recombinant protein encoded by the viral vector component is determined).

The invention therefore provides a method of determining the infectivity of a viral vector vaccine composition comprising: (i) infecting a cell culture with the viral vector vaccine composition; and (ii) quantifying the intracellular level of a protein of a viral vector component of the viral vector vaccine composition in the cell culture by mass spectrometry. The invention also provides a method of potency testing a viral vector vaccine composition comprising: (i) infecting a cell culture with the viral vector vaccine composition; (ii) quantifying the intracellular level of a protein of a viral vector component of the viral vector vaccine composition in the cell culture by mass spectrometry; and (iii) quantifying the level of expression of a recombinant protein encoded by the vaccine composition by mass spectrometry. Here, the same MS analysis can provide the results for both (ii) and (iii). In some embodiments, the protein from the viral vector component that is quantified to determine infectivity is a capsid protein. For example, when an adenovirus vector is used, the capsid protein is Hexon, VII or IX (*e.g.* IX1, IX2, or IX8). The methods described herein can also be used for individually testing the infectivity of single components of multivalent compositions before they are mixed to form the multivalent composition. In some embodiments, the cell culture is composed of cells in which the viral vector vaccine cannot replicate.

Information on the infectivity of a viral vector vaccine composition may be obtained by determining the absolute level of a protein of a viral vector component (like a capsid protein), *e.g.* µg per cell or µg per *y* number of cells (where *y* can be the total number of cells in the cell culture) using the methods of the invention. If the amount of copies of the capsid protein per virus particle is known, then the number of virus particles per cell may be determined. The protein level of the viral vector component that is quantified to determine infectivity may be compared with the level of a cellular protein.

Useful infectivity information may also be obtained from determining the ratio of the amount of viral vector components that are added to the cell culture and the amount of viral vector component that actually infected cells in the culture. Thus, in some embodiments, the method of determining the infectivity of a viral vector vaccine composition involves comparing the intracellular protein level of the viral vector component (e.g. a capsid protein) with the total level of that protein in the cell culture (which may be calculated from the number of viral particles added to the cell culture at the infection stage). Hence, in these embodiments, the method of the invention further comprises the step of quantifying the level of a protein of a viral vector component of the viral vector vaccine composition in the cell culture by mass spectrometry.

The cell culture is typically of mammalian cells as the intended subject for the nucleic acid vaccine or vector vaccine is a mammal (typically a human). Suitable mammalian cells include hamster, cattle, primate (including humans and monkeys) and dog cells. In particular embodiments, the cells are human cells. Various cell types may be used, such as kidney cells, fibroblasts, retinal cells, lung cells, *etc.* In some embodiments, the cells are stable mammalian cells, such as a human cell line (*e.g.* A549 cell line). In some embodiments, the cell culture is composed of cells in which the viral vector vaccine or nucleic acid vaccine cannot replicate. For example, if the viral vector vaccine is replication deficient, the cells of the cell culture should not contain genes which complement the deficient replication machinery of the cell culture. As shown in Example 1, the invention can successfully analyse the potency of expression of an HIV protein from an adenoviral vector vaccine composition comprising a blend of three different adenoviral vector vaccine components (one encoding Env, the second encoding MoslGagPol and the third encoding Mos2GagPol) when a human cell line is infected.

The cell culture is typically infected with a pre-determined amount of the nucleic acid or viral vector vaccine composition. This pre-determined amount is typically an amount that provides useful readings of the expression levels of the proteins of interest within the dynamic range of the mass spectrometer. This amount can be determined based on, *e.g.* a calibration curve using a series of known amounts (*e.g.* a serial dilution) of the nucleic acid or viral vector vaccine composition. For example, for viral vector vaccine compositions, the cell culture is typically infected with a pre-determined multiplicity of infection (MOI) value. This pre-determined MOI value can be determined based on a standard curve generated from the level of the recombinant protein expressed when the cell culture is infected with vaccine compositions in a series of known MOI values. In some embodiments, the viral vector vaccine composition is used to infect the cell culture at an MOI value of: 100 or more, 200 or more, 500 or more, 1000 or more, 5000 or more, 10 000 or more, 25 000 or more or 50 000 or more.

Typically, during the manufacture of a vaccine composition, the vaccine composition is prepared as a bulk composition, and the bulk is then diluted into the final product. The methods of the invention may therefore involve potency testing a sample comprising the vaccine composition, wherein the sample is from: (i) any stage during manufacture of the vaccine, (ii) a bulk before dilution into the final product, or (iii) the final product.

The invention also provides a method of manufacturing vaccine doses, comprising: (i) assaying the relative potency of the vaccine in the bulk as described above; and, if the results of step (i) indicate an acceptable relative potency, (ii) dispensing the bulk vaccine into doses.

The invention also provides a process for analysing a batch of vaccine, comprising : (i) assaying the relative potency of the vaccine from a batch as described above; and, if the results of step (i) indicate an acceptable relative potency, (ii) releasing vaccine from the batch for *in vivo* use.

A test for relative potency can be carried out multiple times in order to determine variance of the assay *e.g.* multiple times (duplicate, triplicate, *etc.*) on a single sample, and/or performed on multiple samples from the same bulk/batch. The invention can involve determining the variation in such multiple assays (*e.g.* the coefficient of variation) as a useful parameter, and in some embodiments the results of the assay are considered as useful only where variation falls within acceptable limits *e.g.* <15%. Sometimes a wider variation is permitted *e.g.* <20%, depending whether tests are performed within

(intra-assay) or in different (inter-assay) experimental sessions. To facilitate determination of relative potency, the expression level should show a linear response to the vaccine composition introduced to the cell culture (*i.e.* dilution of the vaccine composition should bring about a corresponding reduction in the amount of detected recombinant protein). Linearity can be assessed by linear regression e.g. to have $R^2 > 0.95$.

The vaccine composition assessed for potency may be monovalent (*i.e.* encode a single recombinant protein) or multivalent (*i.e.* encode more than one recombinant protein). In some embodiments, the vaccine composition is multivalent. A vaccine composition can be multivalent because it contains different components each of which encodes a different recombinant protein (*e.g.* a first vaccine component encoding protein A, a second vaccine component encoding protein B, a third vaccine component encoding protein C, and so on), or because it contains copies of one component, but that single component encodes different recombinant proteins (*e.g.* a vaccine component encoding proteins A, B and C). In some embodiments, the vaccine composition is vector vaccine composition, for example a viral vector vaccine composition, such as that containing an adenovirus vector vaccine component or a poxvirus vector vaccine component. In some embodiments, the vaccine composition is a multivalent adenovirus vector vaccine composition. Such a vaccine composition may comprise multiple adenovirus vector vaccine components encoding different recombinant proteins and/or an adenovirus vector vaccine component that encodes more than one recombinant protein. Particular viral vectors are discussed below, as are exemplary recombinant proteins that might be encoded by the vectors.

### *Recombinant protein for quantification*

In a cell-based recombinant expression system, there are two broad locations where the recombinant protein may accumulate: as part of the cells or outside the cells (*i.e.* in the culture medium). Inside the cell, the protein may be a soluble protein in the cytoplasm, or a membrane protein, *etc.* The expressed recombinant protein for analysis by a method of the invention can be harvested from the cell according to known methods in the art.

In embodiments where the recombinant protein accumulates as part of the cells, as is typical for the proteins encoded by nucleic acid vaccine components and viral vector vaccine components, the cells are lysed and the cell lysate is harvested. Typically, the cell lysate contains a matrix of proteins, including the recombinant protein and any other proteins expressed from the recombinant expression system, and cellular proteins from the cell. This lysate is a crude cell lysate.

In some embodiments, the cells are harvested under denaturing conditions. In some embodiments, severe denaturing conditions are used to ensure full solvation of all cellular proteins, including membrane proteins. Such denaturing conditions for harvesting cells are known in the art, *e.g.* 2% SDS and 8M urea. The dissolved proteins can then be separated from the cellular debris, to form a clarified cell lysate. Any suitable techniques can be used for this, *e.g.* centrifugation. The resulting clarified protein solution (supernatant) is used in the MS analysis using the techniques described above.

In embodiments where the recombinant protein is secreted from the cell, the supernatant of the cell culture is collected. The soluble proteins are separated from the cellular debris, e.g. by centrifugation. The resulting clarified protein solution (supernatant) is used in the MS analysis using the techniques described above. To facilitate processing, the culture supernatant may be subjected to an optional concentration step.

## *Quantifying the level of a recombinant protein using MS*

The invention uses a MS-based approach to evaluate the expression levels of proteins in recombinant expression systems, such as a cell culture into which a nucleic acid vaccine component or composition or a viral vector vaccine component or composition has been introduced. The invention can be applied to any recombinant protein expressed by any recombinant expression system, and it will be immediately apparent how the methods discussed herein can be applied to recombinant expression systems in general. The principles of the MS-based approach used in the invention are explained below.

Every protein has a unique amino acid sequence, and when the protein is cleaved into peptides by a peptidase (*e.g.* trypsin, chymotrypsin, or others), it yields a set of peptide fragments which can be identified and quantified by mass spectrometry. Some of the peptide fragments produced when a protein is digested have a sequence that is unique to the protein. Accordingly, when that peptide is detected, it can be inferred that the protein it is derived from was present in the sample being analysed. Peptides that can be used to identify the proteins from which they are derived are called proteotypic peptides. Ideally, the proteotypic peptides should also be reliably observed over multiple experiments. Each protein typically has a number of such these identifier peptides. Thus, by monitoring the mass, retention time and fragmentation behaviour of proteotypic peptides which uniquely identify a single protein, the amount of that protein in the sample being analysed can be assessed. By breaking the sequence of the proteins down into peptides, proteins with high sequence identity to one another can be more easily distinguished, as MS can be used to focus on those peptides that comprise the sequence differences.

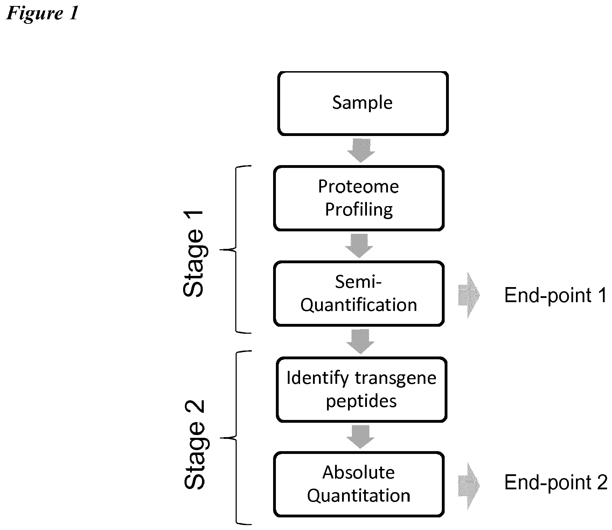

The process of quantifying a recombinant protein using mass spectrometry according is summarised in Figure 1. The quantification can comprise two stages: Stage 1 and Stage 2.

Stage 1 is a semi-quantitative method, which provides an estimate of a recombinant protein's expression based on the relative expression of the recombinant protein compared to the other proteins in the sample, for instance the level of expression of proteins encoded by a nucleic acid vaccine or a viral vector vaccine. Stage 1 typically comprises the steps of: (a) identifying proteins in a sample for mass spectrometry analysis; and (b) determining the relative expression level of each identified protein, including the recombinant protein.

Stage 2 provides an absolute quantification of a recombinant protein expression. Typically, Stage 2 is carried out after Stage 1, because Stage 1 identifies which proteotypic peptides are the best representatives of the recombinant protein, and these peptides can then be analysed further in Stage 2. Stage 2 typically comprises the steps of: (c) detecting a proteotypic peptide of the recombinant protein; and (d) determining the absolute expression level of the recombinant protein by comparing the detected amount of the proteotypic peptide to a known standard.

Hence, in an embodiment of the invention where the relative expression level of a recombinant protein is determined, steps (a) and (b) are carried out. In an embodiment of the invention where the absolute expression level of a recombinant protein is determined, all the steps (a)-(d) are typically carried out, but sometimes only steps (c) and (d) are carried out.

## *Stage 1*

This stage is a bottom-up proteomics approach, where as many peptides as possible in the entire sample are identified. The data from the MS analysis are then used to establish the relative expression level of each identified protein, including the recombinant protein(s) of interest. In this way, a relative expression level of the recombinant protein(s) can be estimated, which is of particular use in potency evaluation of certain vaccines as discussed elsewhere herein. Stage 1 is particularly useful for comparing the expression behaviour of different batches of vaccine, for example between different batches of nucleic acid vaccines or viral vector vaccines.

*(a) Identifying proteins in a sample*

This step makes use of high speed bottom-up proteomics technology to achieve a high coverage of the peptide identifications in the sample under investigation. This step enables the identification of all or substantially all of the proteins in the sample.

Various tandem mass spectrometry (MS/MS) instruments are suitable for this step. The mass spectrometer can be an ion trap, or any other instrument of suitable mass resolution and sensitivity and provides high throughput peptide sequencing. In some embodiment, an orbital ion trap mass analyser, such as an Orbitrap™ [6], or a similar instrument, is used. In some embodiments, a Q-Exactive Orbitrap™ (Thermo Scientific), can be used. Alternatively, time of flight mass spectrometry (TOF) instruments can be used, such as ESI-Q-TOF instruments and others. The Q-Exactive Orbitrap™ instrument is a commercially available instrument that is particularly useful with the invention. Typically, the sample is subjected to peptidase digestion prior to MS analysis, after preparation as explained above. This is to generate peptides amenable to MS analysis. The sample may then be used directly for MS analysis without further enrichment and/or the use of immune reagents. A more detailed discussion of what can be used as the sample for this analysis is discussed below. Accordingly, in some embodiments, the method comprises digesting the sample with a peptidase before the MS analysis step. In some embodiments, a cell lysate is digested with peptidases (*i.e.* no purification and/or concentration steps are undertaken between cell lysis and digestion other than those necessary to generate a clarified lysate, *e.g.* no resin chromatography or affinity purification steps). In some embodiments, a clarified cell lysate is prepared by centrifuging the crude cell lysate and taking the supernatant. The clarified lysate is subjected to peptidase digestion prior to MS analysis.

Any suitable digestion protocol can be used. Suitable peptidases include any one or a combination of the list consisting of: trypsin, endoproteinase LysC, endoproteinase LysN and chymotrypsin. A combination of endoproteinase LysC and trypsin is a useful combination of peptidases. Accordingly, in some embodiments, the method comprises digesting the sample with multiple peptidases before MS analysis, such as at least 2, at least 3, at least 4 or at least 5 peptidases. In some embodiments, the sample is digested with two peptidases, e.g. endoproteinase LysC followed by trypsin. In some embodiments, multiple experiments can run in parallel, with samples separately digested by different peptidase(s).

The processing steps (lysis, peptidase digestion, *etc.*) that prepare the sample for analysis by MS can be automated. The processing can be performed in a 96-well plate format. This high-throughput screening approach allows the automated analysis of multiple samples, such as may occur when running replicate samples of a batch of vaccine composition, or testing different vaccine compositions, or determining the optimal amount of the nucleic acid or viral vector vaccine composition for introducing into cell culture (*i.e.* determining the optimum MOI or MOI range).

The peptides resulting from digestion by the peptidases may be separated by chromatography prior to MS analysis, because this improves the resolution for identifying the peptides in the sample.

For example, liquid chromatography is particularly suitable for this. Accordingly, the method in some embodiments further comprises performing a separation chromatography step. In some embodiments, the chromatography step is liquid chromatography, for example high-performance liquid high-performance liquid chromatography (HPLC) or ultra-performance liquid chromatography (UPLC). In some embodiments reverse phase HPLC is performed to separate the peptides. The mass spectrometer can be directly coupled to a liquid chromatography instrument, *e.g.* a nano-flow UPLC instrument. A nano-UPLC (Thermo Ultimate3000) coupled directly to the ESI source of a Thermo Q-Exactive Orbitrap mass spectrometer is particularly useful with the invention.

Typically, the sample dissolved in an appropriate solvent is loaded onto a reverse phase column (*e.g.* a C18 column). The column is then washed, and the peptides are eluted using an elution buffer. Appropriate buffers and conditions can be determined and optimised according to standard techniques in the art. The peptides are then ionised, for example by electrospay ionisation (ESI), and the masses of the ionised peptides are detected.

The proteins in the sample are identified by the MS spectra to the extent possible using a standard database search. Any suitable database search algorithm can be used, *e.g.* Sequest [7] or MASCOT [8].

Typically, the MS spectra are searched against databases containing the sequences of the proteins that are likely to be present in the sample. These include the sequences of the recombinant protein(s) and other proteins of the recombinant expression system that is used to express the protein, *e.g.* the entire proteome of the cell; if one or more peptidases have been used to digest the sample, the sequences of those peptidases. It is also standard in the art to include common contaminant proteins. For example, if the recombinant protein(s) is expressed in human cells, the sequences of common non-human contaminant proteins are included.

Many protein sequences and proteome databases are available in the art. For example, high quality databases can be obtained from UniProt/SwissProt. These databases undergo constant review, and so are up-to-date. Alternatively, databases can be compiled using routine procedures, such as extracting data from the NCBI databases.

False discovery rates may also be estimated by a suitable algorithm, such as Percolator [9]. Typically, the data are filtered to a suitable level of false positives, *e.g.* 1% at the identified MS/MS spectrum level.

*(b) Determining the relative expression level of the identified proteins*

To obtain the relative expression levels of each identified protein, the MS spectra data characteristics can be converted to represent semi-absolute expression levels. Multiple methods can be used as are common in the art, *e.g.* the ratio of identified MS/MS spectra for a protein over its molecular weight. These ratios can be subsequently normalised to the sum of all ratios in the dataset and expressed in parts per million (ppm).

For example, data from the mass spectra may be used to build a relative expression index of each identified protein based on the following formula. The Relative Expression may be calculated by taking the amount of identified spectra (SC) for a given protein *i,* divided by the molecular weight of protein *i,* normalised by the sum of all such quotients for each protein in the dataset, and expressed as fraction of 1,000,000 ppm (see Reference [10]):

$$Relative\ Expression = \frac{\frac{SCi}{MWi}}{\Sigma(\frac{SCk}{MWk})}\ x\ 1,000,000\ ppm$$

Other forms of semi-absolute quantitation based on other spectral features (*e.g.* MS intensity, MS/MS intensity, unique peptide count, spectral counts, iBAQ *etc.,* see for example references 10,11,12,13,14) and other filter and normalization procedures (*e.g.* those based on Top3 [15], TopN [16] or total intensity per protein) can also be used.

The relative expression of a recombinant protein can be determined by comparing its expression to other proteins in the sample, *e.g.* a cellular marker protein. Relative expression of multiple recombinant proteins can also be determined by comparing their expression levels against each other. Crucial potency information can thus be obtained for comparing batches, culture conditions, infection rates *etc.* that can feed back into the process used for the production of the expression construct (*e.g.* nucleic acid vaccine or viral vector vaccine) itself, *e.g.* production cell type, growth conditions *etc.*

The information generated in stage 1 is consistent and robust, but is to some extent influenced by a protein's ability to be cleaved by a peptidase into ample peptides amenable for MS-based sequence analysis. In several instances this may be sufficient. However, when comparing the expression of multiple recombinant proteins, or when a more accurate expression estimation is required, stage 2 of the method can performed in addition to stage 1.

*Stage 2*

In stage 2, the data obtained in stage one is mined for the specific proteotypic peptides which unambiguously identify a recombinant protein of interest. These peptides can then be used to develop a MS-based assay for absolute quantitation (*e.g.* in ng/cell, or ng/mL) of protein expression. Stage 2 is particularly useful for comparing the expression of multiple recombinant proteins, or when more accurate quantitative information is required, a triple quadrupole instrument is useful for this stage.

Stage 2 provides absolute quantification of a recombinant protein of interest. As explained above, the steps of this stage typically comprise: (c) detecting a proteotypic peptide of the recombinant protein; and (d) determining the absolute expression level of the recombinant protein. These steps are explained in further detail below.

*(c) Detecting a proteotypic peptide of the recombinant protein*

The detailed sequence information generated in stage 1 is mined. The aim at this step is to identify proteotypic peptides which are unique only to a recombinant protein of interest (*i.e.* the sequences of which are not present in any other protein present in the sample).

The exact peptide identifications which lead to the identification of the gene of interest are scrutinised for intensity, reproducibility and fragmentation behaviour. For example, peptides with certain intensities may be selected. In some embodiments, proteotypic peptides having the highest intensities are selected (*e.g.* >1e$^5$ counts per scan in an orbital ion trap mass analyser), and hence these peptides may be most robust and are likely to be detected repeatedly. Sometimes, peptides with certain residues (*e.g.* methionine residues) and/or peptides with certain sequences (*e.g.*

glycosylation consensus sequences) may be omitted. Peptides may also be selected based on length, *e.g.* peptides shorter than 7 and longer than 25 amino acids may be omitted. These characteristics can be screened manually, or using software packages, such as Skyline.

Once the unique proteotypic peptides are identified, they can be used to generate a MS-based assay for absolute quantitation. For this, a triple quadrupole or quadrupole ion trap mass spectrometer is typically used.

The initial step is to set up the instrument in such a way that it only monitors the proteotypic peptides of interest. This is called single reaction monitoring (SRM), or sometimes referred to as multiple reaction monitoring (MRM). This methodology has been extensively developed to quantify small molecules [17,18] and proteins in complex matrixes [19,20,21]. It has the benefit of being sensitive, highly selective and easily multiplexed.

In the SRM operation mode, the first quadrupole ($Q_1$) is set to monitor the intact mass of each proteotypic peptide, the second ($Q_2$) is used as a collision cell to fragment the peptide and in the third ($Q_3$), only a select set of characteristic fragment ions are monitored. The combination of a precursor mass in Q1 and a predicted fragment mass in Q3 is known as a transition, and a set of transition serves to identify the protein from which the fragment is derived.

*(d) Determining the absolute expression level of the recombinant protein*

Once the detection and monitoring of the proteotypic peptides of interest is established, peptide quantitation techniques such as the isotope dilution approach can be used. This is a well-established technique in the art [22,23]. The isotope dilution approach relies on the addition of a known concentration of isotope labelled peptide homologous to the peptide of interest. "Heavy" versions (*e.g.* $^{13}$C, $^{15}$N, *etc.*) of lysine or arginine amino acid residues are incorporated during the peptide synthesis producing a mass difference of 8 and 10 Da, respectively, with the endogenous form of the peptide. Hence, the "heavy" peptides are chemically identical and will behave identically in terms of chromatography, ionization and fragmentation. This means they are useful quantitative internal standards for the proteotypic peptides under investigation. The only difference between the heavy peptides is their precursor mass (in $Q_1$) and their fragment masses (in $Q_3$), so they can be monitored separately from the "light" version originating from the recombinant protein in the sample.

"Heavy" and "light" versions of the peptides elute simultaneously when separated by reversed phase liquid chromatography allowing a concomitant detection and quantification with limited bias due to ionization or interferences from the sample matrix. The "heavy" to "light" peptide peak area ratio is then measured to calculate the peptide concentration in the sample. This approach calculates the peptide concentration but does not provide any information about the precision of measurement over the concentration range of the assay and the linearity of the detector response. Thus, if the sample is spiked with a known concentration of a "heavy" peptide, the amount of recombinant protein in the sample can be determined in an absolute manner by reference to the output signal of the "heavy" peptide.

In order to enable absolute quantitation the proteotypic peptides chosen to be detected in step (c) should therefore also be amenable to chemical synthesis, so that the "heavy" peptides necessary to act as the standard for quantitation can be made.

**Viral vector vaccines**

The methods of the invention find particular use in quantifying expression from and assessing the potency of viral vector vaccines compositions or components. Many different viruses have been adopted for use as vectors for this purpose, and the method of the invention can be applied to all of them.

*Adenovirus vector*

The viral vector may be an adenovirus vector (rAd). Adenovirus vectors are powerful inducers of cellular immune responses and have therefore come to serve as useful components in viral vector vaccines, and have been used to encode protein antigens from lentiviruses and filoviruses, as well as other non-viral pathogens [*e.g.* see 58,59,24,25,26,27].

The adenovirus vector vaccine component can be derived from a human adenovirus, or from an adenovirus that infects other species, including a bovine adenovirus (*e.g.* bovine adenovirus 3, BAdV3), a canine adenovirus (*e.g.* CAdV2), a porcine adenovirus (*e.g.* PAdV3 or 5), or a simian adenovirus (which includes a monkey adenovirus and an ape adenovirus, such as a chimpanzee adenovirus). In some embodiments, the adenovirus vector vaccine component is from a human adenovirus (HAdV, or AdHu) or a simian adenovirus such as chimpanzee adenovirus (ChAd, AdCh, or SAdV). The sequences of most of the human and non-human adenoviruses are known, and others can be obtained using routine procedures.

Examples of adenovirus vector vaccine components that can be assessed using the invention include recombinant adenovirus from serotype 26 (rAd26) or serotype 35 (rAd35) [28], but any adenovirus serotype can be assessed using the methods of the invention.

A packaging cell line is typically used to produce sufficient amounts of adenovirus vector vaccine component. A packaging cell is a cell that comprises those genes that have been deleted or inactivated in the replication-defective vector that encodes the protein to be expressed. Thus all of the protein subunits of the virus are expressed in the packaging cell, allowing the viral particles to assemble and incorporate the replication defective vector into virus particles, thereby forming viral vector vaccine components. Suitable cell lines include, for example, PER.C6, 911, 293, and EI A549.

A wide variety of proteins can be expressed from the adenovirus vectors. Typically, the gene of interest is cloned into the E1 and/or the E3 region of the adenoviral genome.

The gene encoding the recombinant protein be under the control of (*i.e.* operably linked to) an adenovirus-derived promoter (*e.g.* the Major Late Promoter) or may be under the control of a heterologous promoter. Examples of suitable heterologous promoters include a CMV promoter CMV-promoter [29], *e.g.* the CMV immediate early promoter, for instance, comprising nt. -735 to +95 from the CMV immediate early gene enhancer/promoter, and an RSV promoter.

Adenovirus vectors, methods for construction thereof and methods for propagating thereof, are well known in the art (*e.g.* see references 30,31,32,33,34,35,36,37,38,39,40,41). Typically, construction of adenoviral vectors involves the use of standard molecular biological techniques [42,43,44].

In some embodiments, the invention quantifies the protein expression level of a recombinant Ebola virus antigen encoded in an adenovirus vector. In some embodiments, the recombinant protein is an Ebola virus antigen selected from the group consisting of: GP, SGP and NP. In some embodiments of the invention, the Ebola virus antigen is selected from the group consisting of: GP the Zaire strain, SGP of the Zaire strain, NP of the Zaire strain, GP of the Sudan strain, SGP of the Sudan strain, NP of the Sudan strain, GP of the Ivory Coast Ebola strain, SGP of the Ivory Coast Ebola strain and NP of the Ivory Coast Ebola strain.

In some embodiments, the invention quantifies the protein expression level of a recombinant HIV antigen encoded in an adenovirus vector. In some embodiments, the HIV antigen is selected from the group consisting of GAG, POL, ENV and NEF.

Other suitable antigens which can be expressed from viral vector vaccines and which can be quantified using the methods of the invention are discussed below.

*Adeno-associated virus vector*

The viral vector may be an adeno-associated virus (AAV) vector. AAV is a virus which is dependent on the presence of another virus in order to replicate (adenovirus and certain herpesvirus). It is a small virus which causes a very mild immune response, indicating a lack of pathogenicity. AAV can infect both dividing and quiescent cells and persist in an extrachromosomal state without integrating into the genome of the host cell.

As the virus is small, the cloning capacity of the vector is relatively limited and most therapeutic genes require the complete replacement of the virus's 4.8 kilobase genome. Large genes are, therefore, not suitable for use in a standard AAV vector.

11 different serotypes of AAV have been reported [45].

*Poxvirus vector*

The viral vector may be a poxvirus vector. Poxviruses have large genomes, they can readily be used to deliver a wide range of genetic material including multiple genes (*i.e.* act as a multivalent vector).

The poxvirus vector can be from avipoxvirus (*e.g.* fowl pox, canary pox, pigeon pox, turkey pox, quail pox), capripoxvirus (*e.g.* sheep or goat poxvirus), orthopoxvirus (*e.g.* vaccinia, cowpox, mousepox (ectromelia), rabbitpox, racoon pox, and monkey pox), or suipoxvirus (*e.g.* swine pox).

The poxvirus may be attenuated (*i.e.* weakened) to reduce virulence by *e.g.* selection or chemical or genetic mutagenesis, e.g. see the NYVAC strain of vaccinia [46] and the MVA strain [47].

Basic techniques for preparing recombinant poxviruses containing a gene of interest sequence are well known in the art. For example, a method involves homologous recombination between the viral DNA sequences flanking the DNA sequence in a donor plasmid and homologous sequences present in the parental virus [48,49]. Other techniques include using a restriction endonuclease site that is naturally present or artificially inserted in the parental viral vector to insert the heterologous DNA [50].

Typically, a gene of interest is inserted into a recombinant poxvirus vector at a site that does not substantially affect virus viability of the resultant recombinant virus. For example, the thymidine kinase (TK) gene is a suitable insertion site. In vaccinia, in addition to the TK region, other insertion sites, *e.g.* the HindIII M fragment can be used. In fowlpox, in addition to the TK region, other insertion sites, *e.g.* the BamHI J fragment, the EcoRI-V7c/III fragment, the EcoRV-HindIII fragment, BamHI fragment or the HindIII fragment can be used [51].

The gene of interest may be under the control of (*i.e.* operably linked to) a poxvirus-derived promoter. For example, a promoter based upon native promoters, such as the vaccinia 7.5K or 40K or fowlpox promoters such as FPV C1 may be used. Enhancer elements can also be used in combination with promoters to increase the level of expression.

### Other vector vaccines

The methods of the invention are also useful in quantifying expression from and assessing the potency of other vector vaccine compositions or components, such as bacterial or yeast vector vaccine compositions or components.

Typically a bacterial vector is derived from a mutant or genetically modified bacterium. The bacterium is optionally attenuated. A number of bacterial species have been developed for use as vector vaccines, *e.g. Shigella flexneri, E. coli, Listeria monocytogenes, Yersinia enterocolitica, Salmonella typhimurium, Salmonella typhi or mycobacterium* [52,53,54,55]. The bacterial vector may be a facultative, intracellular bacterial vector. Preparations of bacteria useful as vector vaccines are known in the art.

A yeast vector may be derived from any yeast strain [56]. In some embodiments, the yeast vector is from a non-pathogenic yeast strain. Yeast vectors can be derived from *S. cerevisiae, C. albicans, H. polymorpha, P. pastoris and S. pombe. S. cerevisiae* is particularly useful as vector vaccines because it is relatively easy to manipulate and is GRAS [57]. Preparations of yeasts useful as vector vaccines are known in the art.

### Gene therapy

Many viral vector vaccine components are replication deficient, and the genes that they encode are only expressed temporarily when administered to a subject. This is useful in situations where only a short exposure to the proteins encoded by the vaccine is necessary, for example to generate an immune response against a protein target as part of a vaccination schedule. However, in some instances, persistent expression of the protein is desired, for example to complement a defective gene in a subject, in a process known as gene therapy. Gene therapy constructs can be tested using the methods of the invention in exactly the same manner as viral vector vaccines as discussed above.

Often, viral vectors are used to introduce the nucleic acid for gene therapy into cells, including the following types of viruses: retrovirus, adenovirus, lentivirus, herpes simplex virus, vaccinia virus, and adeno-associated virus. For long term treatment, the nucleic acid contained in the viral vector is targeted for incorporation into the chromosomes of the cells of the subject being treated (and therefore into the chromosome of any cells to which the gene therapy construct is tested on as part of its potency testing). Apart from this, however, the potency testing can be performed as for viral vaccine vector compositions and components, as discussed above.

### Exemplary features of the invention

The invention provides a method for determining the level of protein expression by a recombinant expression system, comprising the step of quantifying a recombinant protein expressed by the system using mass spectrometry, optionally wherein the recombinant expression system is a cell culture and wherein the cell culture is lysed to form a cell lysate that is analysed by mass spectrometry to quantify the recombinant protein, optionally wherein the cell lysate is a clarified cell lysate. The recombinant expression system can be a cell culture (i) infected with a viral vector vaccine composition encoding the recombinant protein or (ii) transformed by a nucleic acid vaccine composition encoding the recombinant protein, optionally wherein the viral vector vaccine composition comprises an adenovirus vector vaccine component encoding the recombinant protein, such as wherein the viral vector vaccine composition encodes at least two recombinant proteins, for example wherein the viral vector vaccine composition comprises (i) an adenovirus vector vaccine component encoding at least two recombinant proteins and/or (ii) multiple different adenovirus vector vaccine components encoding different recombinant proteins. Sometimes the viral vector vaccine composition comprises a poxvirus vector vaccine component encoding the recombinant protein. Sometimes two or more recombinant proteins are quantified using mass spectrometry. Sometimes another protein in the recombinant expression system has at least 85% identity to a recombinant protein. The method can further comprise the step of introducing the gene encoding the recombinant protein into a cell culture to generate a recombinant expression system and expressing the recombinant protein, prior to the step of quantifying the recombinant protein by mass spectrometry, optionally wherein the quantifying is determining the relative or the absolute expression level of the recombinant protein optionally , wherein the determining is: (i) determining the relative expression level of the recombinant protein comprises the steps of: (a) identifying proteins in a sample by mass spectrometry analysis; and (b) determining the relative expression level of each identified protein, including the recombinant protein, optionally wherein the relative determination is performed by determining expression of the recombinant protein relative to a cellular marker protein; or (ii) determining the absolute expression level of the recombinant protein comprises the steps of: (a) identifying proteins in a sample by mass spectrometry analysis; (b) determining the relative expression level of each identified protein, including the recombinant protein; (c) detecting a proteotypic peptide of the recombinant protein; and (d) determining the absolute expression level of the recombinant protein by comparing the detected amount of the proteotypic peptide to a known standard.

### *Exemplary vaccine encoded recombinant proteins detectable by the invention*

Nucleic acid and viral vectors are most frequently used to express an antigen, or an antigenic determinant thereof, which is capable of eliciting an immune response in a subject (*i.e.* nucleic acid and viral vectors vaccines as discussed above). This antigen may be from an infectious agent, or may be characteristic of a tumour. Sometimes, however, the vectors are designed to encode other proteins to achieve other therapeutics aims. Similarly, the proteins encoded by gene therapy constructs differ from those encoded by vaccines as the aim for these constructs is to provide stable long term expression. Here therefore, often the recombinant protein is a cellular enzyme which is deficient in the subject to be treated.

#### *Infectious agents*

The antigen can be derived from a bacterium, a virus, yeast or a parasite, which can cause infectious disease.
For example, the antigen may be from malaria-causing organisms, such as *Plasmodium falciparum,* tuberculosis-causing organism such as *Mycobacterium tuberculosis,* yeasts, or viruses. Antigens from viruses, such as flaviviruses (*e.g.* West Nile Virus, Hepatitis C Virus, Japanese Encephalitis Virus, Dengue Virus), Ebola virus, Human Immunodeficiency Virus (HIV), and Marburg virus may be used.
The following examples of antigens may be of particular interest for the present invention.
An Ebola virus antigen selected from the group consisting of: GP, SGP and NP [58,59,60]. These antigens can be of the Zaire, Sudan or Ivory Coast Ebola strain.
An antigen from HIV, such as gag, pol, env, nef, or variants thereof [61,62,63,64,65].
An antigen from *M. tuberculosis, e.g.* the Ag85A, Ag85B and/or the TB10.4 proteins.
An antigen from an influenza virus, such as HA, NA, M, or NP protein [66,67,68].
An antigen from a measles virus, e.g. HA protein [69].
An antigen from a rabies virus glycoprotein [70].
An antigen from malaria, poliovirus, tetanus (e.g. tetanus toxoid), diphtheria (e.g. diphtheria toxoid), or *Bordetella pertussis* or respiratory syncytial virus (RSV).

#### *Tumour antigens*

A developing area relates to the use of vaccines to stimulate the body's immune system to find and kill cancer cells. A tumour antigen nucleic acid or viral vector vaccine component therefore encodes tumour antigens (typically proteins or variants of proteins) which are expressed on tumour cells but not on normal cells. The aim of this approach is to prime the immune system to attack the cancer cells (usually in conjunction with the administration of other agents to boost the immune response, such as an anti-CTLA4 antibody).
Exemplary tumour antigens include TAG-72, BING-4, Calcium-activated chloride channel 2, Cyclin-B1, Ep-CAM, EphA3, Her2/neu, telomerase, mesothelin, SAP-1, surviving, NY-ESO-1/LAGE-1, PRAME, SSX-2, Melan-A/MART-1, Gp100/pmel17, tyrosinase, TRP-1/-2, MC1R, β-catenin, CDK4, CML66, MART-2, p53, Ras, TGF-βRII and MUC1.

#### *Proteins for gene therapy*

Typical recombinant proteins include adenosine deaminase, Factor IX, beta-globin, lipoprotein lipase, dopamine, and IL2RG.

### *Recombinant expression systems for the production of proteins*

As noted above, the method of the invention is particularly useful for quantifying expression of nucleic acid vaccines and vector vaccines, but can be applied to quantitate recombinant protein expression by any recombinant expression systems.
Recombinant expression systems are usually used to produce proteins which are subsequently used for other purposes (as opposed to acting as a proxy to test the effectiveness of the genetic construct encoding the recombinant protein *in vivo,* as in the case of nucleic acid vaccines or vector vaccines as discussed elsewhere herein). Of greatest commercial importance today are recombinant expression systems that are used for the production of proteins, for example biopharmaceuticals such as antibodies. Particular biopharmaceuticals that can be detected using the method of the invention are discussed below.

Accordingly, one difference between the quantification of recombinant protein expression in production expression systems versus potency testing systems as discussed above is that a much wider range of expression hosts can be used (typically, in potency testing assays, the cell type will be from a human or mammal to which the nucleic acid vaccine/viral vector vaccine is meant to be administered). Commonly used protein expression systems include those derived from bacteria, yeast, baculovirus/insect, and mammalian cells. Recombinant expression systems may be cell-based or cell-free, but are most commonly cell-based.

There are many ways in which a production recombinant expression system can be set up, as discussed below, and protein expression by all of these different systems can be assessed by a method of the invention. Recombinant expression includes use of a naturally occurring combination of promoter and gene, but expression from a non-naturally occurring context, for example, a human gene in combination with its promoter as found in the human genome, but placed on an autonomously replicating expression plasmid or on a circular or linear genetic element that does not replicate in the cell (for example as is often the case with transient expression systems). The promoter may be switched for one which is a strong constitutive promoter to maximise expression of the protein. In some instances, the combination of gene and promoter may be integrated into the genome of a cell so that the cell expresses the gene. Sometimes the combination of gene and promoter in the chromosome is achieved by inserting a non-native promoter into the genome of a cell at a location next to a gene already in the chromosome to drive its expression. Sometimes, a non-naturally occurring gene is inserted next to a promoter in the genome of the host cell, such that the promoter can drive expression of the gene.

The gene may be modified so that the protein expressed from it (the recombinant protein) has a different property compared to the wildtype protein, *e.g.* different activity, different expression level and/or different conformation. This can be achieved by common techniques in the art, such as altering the nucleic acid sequences that code for the protein and/or the transcriptional regulatory elements that regulate the expression of the protein. For example, these sequences can be modified or replaced. For example, the coding sequence can be modified, *e.g.* codon-optimised to ensure proper expression in the cell in which the gene is to be expressed. Codon-optimisation is a technology widely applied in the art. In some embodiments, the coding sequence may be mutated so that the recombinant protein is less toxic.

Typically, cell-based expression systems are used for the expression of these proteins, *e.g.* mammalian cells, such as CHO cells, COS cells, NS0 cells or human cell lines (*e.g.* HEK293 cells). Examples of proteins such these include insulin, antibodies, receptor fusion protein, EPO *etc.* A fuller list of exemplary proteins which can be expressed in production recombinant expression systems and analysed by the method of the invention is set out below.

The desirable characteristics of recombinant expression systems for the production of biopharmaceuticals are high productivity and stability. The methods of the invention are therefore useful in measuring expression of recombinant proteins by production recombinant expression systems because the methods can determine the relative production of the protein of interest in comparison to other cellular proteins, and so provide a good measure of the productivity of the cell. The methods are also particularly useful where the protein being produced is a multicomponent protein, such as an antibody. Here, because the expression level of the individual subunits of the protein can be monitored individually, it may be possible to alter the expression of the individual components so that they are produced in the correct ratios to maximise the expression of the protein being produced. This may be useful when the recombinant protein is an antibody, which is made up of separately transcribed and translated light chain and heavy chain subunits. MS-based methods also have the advantage that they can monitor glycosylation and other post-translational changes in protein structure (*e.g.* post-translational deamidation of asparagine or aspartate isomerisation [71,72]).

The particular form of the nucleic acid which encodes the recombinant protein detected by the method of the invention is not important. The nucleic acid may be circular or linear. The nucleic acid molecule comprising the nucleic acid sequence which encodes the recombinant protein is commonly called an expression vector.

The expression vector may be a virus vector. Viral vectors are more commonly used in those embodiments relating to the potency testing of recombinant expression constructs like nucleic acids vaccines or vector vaccines, and so are discussed in more detail above, but also have an application in inducing transient expression in other cell culture techniques, or as a means for introducing a gene encoding a recombinant protein into the genome of a cell.

The expression vector may be a non-viral vector. Examples of non-viral vectors used in microbiology include plasmid vectors, *e.g. Escherichia coli*-derived plasmids (ColE-series plasmids such as pBR322, pUC18, pUC19, pUC118, pUC119, pBluescript), *Actinomyces*-derived plasmids (*e.g.* pIJ486), *Bacillus subtilis*-derived plasmids (*e.g.* pUB110, pSH19), yeast-derived plasmids (*e.g.* YEp13, YEp 24, Ycp50), and artificial plasmid vectors. Examples of useful eukaryotic vectors for the expression of recombinant proteins, e.g. mAbs, include pcDNAI and pcDM8 (manufactured by Funakoshi Corporation), pAGE107 [73], pAS3-3 [74],pcDNAI/Amp (manufactured by Invitrogen), pcDNA3.1 (manufactured by Invitrogen), pREP4 (manufactured by Invitrogen), pKANTEX93 [75], pCI, pAdVAntage, pCMVTnT and pTarget vectors (all from Promega).

If the expression vector is autonomously replicable outside the chromosome (*i.e.* containing an origin of replication), the vector may incorporate a selectable marker to ensure that the plasmid is not lost from the cell culture over time. Examples of the selectable marker include the dihydrofolate reductase (DHFR) gene, the glutamine synthetase

gene, or the *Schizosaccharomyces pombe* TPI gene, and genes for resistance to drugs such as ampicillin, kanamycin, tetracycline, chloramphenicol, neomycin, or hygromycin. Other suitable markers will be immediately apparent to the one of skill in the art.

Examples of suitable mammalian host cell lines include the COS cell of monkey kidney origin, mouse L cells, murine C127 mammary epithelial cells, mouse Balb/3T3 cells, Chinese hamster ovary cells (CHO), human 293 EBNA and HeLa cells, myeloma, and baby hamster kidney (BHK) cells.

Expression constructs can be introduced into cells using any technique known to those of skill in the art, *e.g.* see Reference 42 and other laboratory manuals. For example, transfection protocols, such as calcium phosphate or calcium chloride co-precipitation, lipofection, electroporation *etc.,* can be used.

As noted above, any recombinant protein expressed from a recombinant expression system may be quantified using the methods of the invention. Hence, the particular recombinant protein is not a critical aspect of the invention.

Common proteins expressed by recombinant expression systems that can be detected by the method of the invention include interleukins, such as IL-1, IL-2, IL-3, IL-4, IL-5, IL-6, IL-7, IL-8, IL-9, IL-10, IL-11, IL-12, IL-13, IL-14, IL-15, IL-17, IL-18, IL-20, IL-21, IL-22, IL-23, IL-24, IL-25, IL-26, IL-27, IL-28, IL-31 and IL-35, and their receptors, TNF$\alpha$, growth factor receptors, such as TNFAlphaR, RGFbetaR, TSHR, VEGFR/VPFR, FGFR, EGFR, PTHrPR, PDGFR family, EPO-R, GCSF-R and other hematopoietic receptors; interferon receptors, Ig receptors, blood factors, such as complement C3b, complement C5a, complement C5b-9, Rh factor, fibrinogen, fibrin, and myelin associated growth inhibitor; enzymes, such as cholesterol ester transfer protein, membrane bound matrix metalloproteases, and glutamic acid decarboxylase (GAD) ; and miscellaneous antigens including ganglioside GD3, ganglioside GM2, LMP1, LMP2, eosinophil major basic protein, eosinophil cationic protein, pANCA, Amadori protein, Type IV collagen, glycated lipids, v-interferon, A7, and Fas (AF0-1) and oxidised-LDL, leukocyte markers, such as CD2, CD3, CD4, CD5, CD6, CD7, CD8, CDlla,b,c, CD13, CD14, CD15, CD19, CD20, CD22, CD23, CD27 and its ligand, CD2S and its ligands B7.1, B7.2, B7.3, CD29 and its ligand, CD30 and its ligand, CD40 and its ligand gp39, CD44, CD45 and isoforms, CD56, CD58, CD69, CD72, CTLA-4, PD-1, PD-L1, PD-L2, LFA-1 and TCR histocompatibility antigens, such as MHC class I or II, the Lewis Y antigens, Slex, Sley, SIea, and Selb; adhesion molecules, including the integrins, such as VLA-1, VLA-2, VLA-3, VLA-4, VLA-5, VLA-6, LFA-1, Mac-1, aV$\beta$3 and the selectins, such as L-selectin, E-selectin, and P-selectin and their counterreceptors VCAM-1, ICAM-1, ICAM-2, and LFA-3; chemokines, such as PF4, RANTES, MIPla, MCPI, IP-10, ENA-78, NAP-2, Gro$\alpha$, Gro$\beta$, and IL-8; growth factors, such as TNFalpha, TGFbeta, TSH, VEGF/VPF, PTHrP, EGF family, FGF, PDGF family, endothelin, Fibrosin, Laminin, and gastrin releasing peptide.

Antibodies against any antigen are suitable for detection by the method of the invention, for example against any of these targets. Particular antibodies that can detected by the invention when expressed by recombinant expression systems include abagovomab, abciximab, abrilumab, actoxumab, adalimumab, adecatumumab, aducanumab, afelimomab, afutuzumab, alemtuzumab, alirocumab, amatuximab, anifrolumab, anrukinzumab, apolizumab, arcitumomab, aselizumab, atinumab, atorolimumab, bapineuzumab, basiliximab, bavituximab, bectumomab, belimumab, benralizumab, bertilimumab, besilesomab, bevacizumab, bezlotoxumab, biciromab, bimagrumab, blinatumomab, blosozumab, briakinumab, brodalumab, canakinumab, caplacizumab, carlumab, catumaxomab, cedelizumab, certolizumab, cetuximab, cixutumumab, clazakizumab, clenoliximab, conatumumab, concizumab, crenezumab, dacetuzumab, daclizumab, dalotuzumab, daratumumab, demcizumab, denosumab, detumomab, dinutuximab, diridavumab, drozitumab, duligotumab, dupilumab, durvalumab, dusigitumab, ecromeximab, eculizumab, edobacomab, edrecolomab, efalizumab, efungumab, eldelumab, elotuzumab, elsilimomab, emibetuzumab, enavatuzumab, enokizumab, enoticumab, ensituximab, epratuzumab, erlizumab, ertumaxomab, etaracizumab, etrolizumab, evinacumab, evolocumab, exbivirumab, fanolesomab, faralimomab, farletuzumab, fasinumab, felvizumab, fezakinumab, ficlatuzumab, figitumumab, flanvotumab, fletikumab, fontolizumab, foralumab, foravirumab, fresolimumab, fulranumb, futuximab, galiximab, ganitumab, gantenerumab, gavilimomab, gevokizumab, girentuximab, golimumab, gomiliximab, guselkumab, ibalizumab, icrucumab, igovomab, imciromab, imgatuzumab, inclacumab, infliximab, intetumumab, inolimomab, ipilimumab, iratumumab, itolizumab, ixekizumab, keliximab, labetuzumab, lambrolizumab, lampalizumab, lebrikizumab, lemalesomab, lerdelimumab, lexatumumab, libivirumb, ligelizumab, lintuzumab, lirilumab, lodelcizumab, lucatumumab, lumiliximab, mapatumumab, margetuximab, maslimomab, mavrilimumab, matuzumab, mepolizumab, metelimumab, milatuzumab, minretumomab, mitumomab, mogamulizumab, morolimumab, motavizumab, namilumab, narnatumab, natalizumab, nebacumab, necitumumab, nerelimomab, nesvacumab, nimotuzumab, nivolumab, obiltoxaximab, ocaratuzumab, ocrelizumab, odulimomab, ofatumumab, olaratumab, olokizumab, omalizumab, onartuzumab, ontuxizumab, oregovomab, orticumab, otelixizumab, otlertuzumab, oxelumab, ozanezumab, ozoralizumab, pagibaximab, palivizumab, panitumumab, pankomab, panobacumab, parsatuzumab, pascolizumab, pateclizumab, patritumab, pembrolizumab, pemtumomab, perakizumab, pertuzumab, pexelizumab, pidilizumab, pintumomab, placulumab, ponezumab, priliximab, pritoxaximab, pritumumab, quilizumab, racotumomab, radretumab, rafivirumab, ramucirumab, ranibizumab, raxibacumab, regavirumab, reslizumab, rilotumumab, rituximab, robatumumab, roledumab, romosozumab, rontalizumab, rovelizumab, ruplizumab, samalizumab, sarilumab, secukinumab, seribantumab, setoxaximab, sevirumab, sibrotuzumab, sifalimumab, siltuximab, simtuzumab, siplizumab, sirukumab, solanezumab, solitomab, sonepcizumab, sontuzumab, stamulumab, sulesomab, suvizumab, tabalumab, tadocizumab, tanezumab, tarextumab, tefibazumab, tenatumomab, teneliximab, teplizumab, teprotumumab, ticilimumab, tildrakizumab, tigatuzumab, tocilizumab, toralizumab, tositumomab, tovetumab, tralokinumab, trastuzumab, tregalizumab, tremelimumab, tuvirumab, ublituximab, urelumab, urtoxazumab, ustekinumab, vantictumab, vapaliximab, varlilumab, vatelizumab, vedolizumab, veltuzumab, vepalimomab, vesencumab, visilizumab, volociximab, votumumab, zalutumumab, zanolimumab, zatuximab, and ziralimumab. Expression of receptor fusion proteins such as entanercept, abatercept *etc.* can also be quantified by the method of the invention.

*General*

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x is optional and means, for example, *x*+10%. Identity between polypeptide sequences is in some embodiments determined by the Smith-Waterman homology search algorithm as implemented in the MPSRCH program (Oxford Molecular), using an affine gap search with parameters *gap open penalty=12* and *gap extension penalty=1.*

**SEQUENCES**

| SEQ ID NO | |
|-----------|---|
| 1. | Env<br><br>MRVTGIRKNYQHLWRWGTMLLGILMICSAAGKLWVTVYYGVPVWKEATTTLFCASDAKAYD TEVHNVWATHACVPTDPNPQEVVLENVTENFNMWKNNMVEQMHEDIISLWDQSLKPCVKLT PLCVTLNCTDDVRNVTNNATNTNSSWGEPMEKGEIKNCSFNITTSIRNKVQKQYALFYKLDVV PIDNDSNNTNYRLISCNTSVITQACPKVSFEPIPIHYCAPAGFAILKCNDKKFNGTGPCTNVSTVQ CTHGIRPVVSTQLLLNGSLAEEEVVIRSENFTNNAKTIMVQLNVSVEINCTRPNNNTRKSIHIGPG RAFYTAGDIIGDIRQAHCNISRANWNNTLRQIVEKLGKQFGNNKTIVFNHSSGGDPEIVMHSFN CGGEFFYCNSTKLFNSTWTWNNSTWNNTKRSNDTEEHITLPCRIKQIINMWQEVGKAMYAPPI RGQIRCSSNITGLLLTRDGGNDTSGTEIFRPGGGDMRDNWRSELYKYKVVKIEPLGVAPTKAKR RVVQSEKSAVGIGAVFLGFLGAAGSTMGAASMTLTVQARLLLSGIVQQQNNLLRAIEAQQHLL QLTVWGIKQLQARVLAVERYLKDQQLLGIWGCSGKLICTTTVPWNASWSNKSLDKIWNNMT WMEWEREINNYTSLIYTLIEESQNQQEKNEQELLELDKWASLWNWFDISNWLW |
| 2. | Mos1GagPol<br><br>MGARASVLSGGELDRWEKIRLRPGGKKKYRLKHIVWASRELERFAVNPGLLETSEGCRQILGQ LQPSLQTGSEELRSLYNTVATLYCVHQRIEIKDTKEALEKIEEEQNKSKKKAQQAAADTGNSSQ VSQNYPIVQNIQGQMVHQAISPRTLNAWVKVVEEKAFSPEVIPMFSALSEGATPQDLNTMLNT VGGHQAAMQMLKETINEEAAEWDRVHPVHAGPIAPGQMREPRGSDIAGTTSTLQEQIGWMTN NPPIPVGEIYKRWIILGLNKIVRMYSPVSILDIRQGPKEPFRDYVDRFYKTLRAEQASQDVKNWM TETLLVQNANPDCKTILKALGPAATLEEMMTACQGVGGPGHKARVLAEAMSQVTNSATIMMQ RGNFRNQRKTVKCFNCGKEGHIAKNCRAPRKKGCWKCGKEGHQMKDCTERQANFLGKIWPS NKGRPGNFLQNRPEPTAPPEESFRFGEETTTPSQKQEPIDKEMYPLASLKSLFGNDPSSQMAPISP IETVPVKLKPGMDGPRVKQWPLTEEKIKALTAICEEMEKEGKITKIGPENPYNTPVFAIKKKDST KWRKLVDFRELNKRTQDFWEVQLGIPHPAGLKKKKSVTVLAVGDAYFSVPLDEGFRKYTAFTI PSTNNETPGIRYQYNVLPQGWKGSPAIFQCSMTRILEPFRAKNPEIVIYQYMAALYVGSDLEIGQ HRAKIEELREHLLKWGFTTPDKKHQKEPPFLWMGYELHPDKWTVQPIQLPEKDSWTVNDIQKL VGKLNWASQIYPGIKVRQLCKLLRGAKALTDIVPLTEEAELELAENREILKEPVHGVYYDPSKD LIAEIQKQGHDQWTYQIYQEPFKNLKTGKYAKMRTAHTNDVKQLTEAVQKIAMESIVIWGKTP KFRLPIQKETWETWWTDYWQATWIPEWEFVNTPPLVKLWYQLEKDPIAGVETFYVAGAANRE TKLGKAGYVTDRGRQKIVSLTETTNQKTALQAIYLALQDSGSEVNIVTASQYALGIIQAQPDKS ESELVNQIIEQLIKKERVYLSWVPAHKGIGGNEQVDKLVSSGIRKVLFLDGIDKAQEEHEKYHS NWRAMASDFNLPPVVAKEIVASCDQCQLKGEAMHGQVDCSPGIWQLACTHLEGKIILVAVHV ASGYIEAEVIPAETGQETAYFILKLAGRWPVKVIHTANGSNFTSAAVKAACWWAGIQQEFGIPY NPQSQGVVASMNKELKKIIGQVRDQAEHLKTAVQMAVFIHNFKRKGGIGGYSAGERIIDIIATDI QTKELQKQIIKIQNFRVYYRDSRDPIWKGPAKLLWKGEGAVVIQDNSDIKVVPRRKVKIIKDYG KQMAGADCVAGRQDED |
| 3. | Mos2GagPol |

| SEQ ID NO | |
|---|---|
| | MGARASILRGGKLDKWEKIRLRPGGKKHYMLKHLVWASRELERFALNPGLLETSEGCKQIIKQ LQPALQTGTEELRSLFNTVATLYCVHAEIEVRDTKEALDKIEEEQNKSQQKTQQAKEADGKVS QNYPIVQNLQGQMVHQPISPRTLNAWVKVIEEKAFSPEVIPMFTALSEGATPQDLNTMLNTVGG HQAAMQMLKDTINEEAAEWDRLHPVHAGPVAPGQMREPRGSDIAGTTSNLQEQIAWMTSNPP IPVGDIYKRWIILGLNKIVRMYSPTSILDIKQGPKEPFRDYVDRFFKTLRAEQATQDVKNWMTD TLLVQNANPDCKTILRALGPGATLEEMMTACQGVGGPSHKARVLAEAMSQTNSTILMQRSNF KGSKRIVKCFNCGKEGHIARNCRAPRKKGCWKCGKEGHQMKDCTERQANFLGKIWPSHKGRP |
| | GNFLQSRPEPTAPPAESFRFEETTPAPKQEPKDREPLTSLRSLFGSDPLSQMAPISPIETVPVKLKP GMDGPKVKQWPLTEEKIKALVEICTEMEKEGKISKIGPENPYNTPIFAIKKKDSTKWRKLVDFR ELNKRTQDFWEVQLGIPHPAGLKKKKSVTVLAVGDAYFSVPLDEDFRKYTAFTIPSINNETPGIR YQYNVLPQGWKGSPAIFQSSMTKILEPFRKQNPDIVIYQYMAALYVGSDLEIGQHRTKIEELRQ HLLRWGFTTPDKKHQKEPPFLWMGYELHPDKWTVQPIVLPEKDSWTVNDIQKLVGKLNWAS QIYAGIKVKQLCKLLRGTKALTEVVPLTEEAELELAENREILKEPVHGVYYDPSKDLIAEIQKQG QGQWTYQIYQEPFKNLKTGKYARMRGAHTNDVKQLTEAVQKIATESIVIWGKTPKFKLPIQKE TWEAWWTEYWQATWIPEWEFVNTPPLVKLWYQLEKEPIVGAETFYVAGAANRETKLGKAGY VTDRGRQKVVSLTDTTNQKTALQAIHLALQDSGLEVNIVTASQYALGIIQAQPDKSESELVSQII EQLIKKEKVYLAWVPAHKGIGGNEQVDKLVSRGIRKVLFLDGIDKAQEEHEKYHSNWRAMAS EFNLPPIVAKEIVASCDKCQLKGEAIHGQVDCSPGIWQLACTHLEGKVILVAVHVASGYIEAEVI PAETGQETAYFLLKLAGRWPVKTIHTANGSNFTSATVKAACWWAGIKQEFGIPYNPQSQGVVA SINKELKKIIGQVRDQAEHLKTAVQMAVFIHNFKRKGGIGEYSAGERIVDIIASDIQTKELQKQIT KIQNFRVYYRDSRDPLWKGPAKLLWKGEGAVVIQDNSDIKVVPRRKAKIIRDYGKQMAGDDC VASRQDED |
| 4. | Env4<br>AIEAQQHLLQL TVWGIK |
| 5. | Mos1.3<br>IGPENPYNTPVFAIK |
| 6. | Mos2.1<br>IGPENPYNTPIFAIK |
| 7. | Mos2.2<br>YTAFTIP SINNETPGIR |
| 8. | Act1<br>SYELPDGQVITIGNER |
| 9. | G3P1 |

| SEQ ID NO | |
|---|---|
| | LISWYDNEFGYSNR |
| 10. | G3P3a<br>AVGKVIPEELNGK |
| 11. | VII1<br>TTVDDVIDSWADAR |
| 12. | Env1<br>EATTTLFZASDAK |
| 13. | Env2<br>VSFEPIPIHYZAPAGFAILK |
| 14. | Env3 and Env3a<br>AFYT AGDIIGDIR |
| 15. | Mos1.1<br>FAVNPGLLETSEGZR |
| 16. | Mos1.2<br>SLYNTVATLYZVHQR |
| 17. | Mos1.4<br>IVSLTETTNQK |
| 18. | Mos2.3 and Mos2.3a<br>IATESIVIWGK |
| 19. | Mos2.4<br>WSLTDTTNQK |
| 20. | Mos2.5<br>FALNPGLLETSEGZK |
| 21. | G3P2<br>VIPELNGK |
| 22. | G3P3a<br>AVGKVIPEELNGK |
| 23. | Hex1 |

| SEQ ID NO | |
|---|---|
| | ATDTYFSLGNK |
| 24. | IX1<br>LLALLAELEALSR |
| 25. | Mos1.Env proteotypic peptide |
| | IEPLGVAPTK |
| 26. | Mos2S.Env<br><br>MRVRGMLRNWQQWWIWSSLGFWMLMIYSVMGNLWVTVYYGVPVWKDAKTTLFCASDAKA YEKEVHNVWATHACVPTDPNPQEIVLGNVTENFNMWKNDMVDQMHEDIISLWDASLEPCVK LTPLCVTLNCRNVRNVSSNGTYNIIHNETYKEMKNCSFNATTVVEDRKQKVHALFYRLDIVPL DENNSSEKSSENSSEYYRLINCNTSAITQACPKVSFDPIPIHYCAPAGYAILKCNNKTFNGTGPCN NVSTVQCTHGIKPVVSTQLLLNGSLAEEEIIIRSENLTNNAKTIIVHLNETVNITCTRPNNNTRKSI RIGPGQTFYATGDIIGDIRQAHCNLSRDGWNKTLQGVKKKLAEHFPNKTIKFAPHSGGDLEITTH TFNCRGEFFYCNTSNLFNESNIERNDSIITLPCRIKQIINMWQEVGRAIYAPPIAGNITCRSNITGLL LTRDGGSNNGVPNDTETFRPGGGDMRNNWRSELYKYKVVEVKPLGVAPTEAKRRVVEREKR AVGIGAVFLGILGAAGSTMGAASITLTVQARQLLSGIVQQQSNLLRAIEAQQHMLQLTVWGIKQ LQTRVLAIERYLQDQQLLGLWGCSGKLICTTAVPWNTSWSNKSQTDIWDNMTWMQWDKEIG NYTGEIYRLLEESQNQQEKNEKDLLALDSWNNLWNWFSISKWLWYIKIFIMIVGGLIGLRIIFAV LSIVNRVRQGY |
| 27. | Mos2s.Env proteotypic peptide<br>TTLF[C]ASDAK - [C] means cysteine was carbamidomethylated after synthesis |

**BRIEF DESCRIPTION OF THE FIGURES**

Figure 1 illustrates the typical method steps of the invention.

Figure 2 shows the mass spectra data of a trivalent adenovirus-based HIV vaccine drug product expressed in A549 cells. "PSMs" corresponds to the MS/MS spectra; "proteins" corresponds to the amount of protein.

Figure 3 shows the three recombinant proteins from an HIV viral vector vaccine composition ("Env", "Mos1GagPol" and "Mos2GagPol") in the context of the A549 proteome. Actin from A549 cells is ranked no. 1 at 7712 ppm; Env is ranked no. 338 at 802 ppm; MoslGagPol is ranked no. 867 at 304 ppm; Mos2GagPol is ranked 902 at 289 ppm; and Hexon from the adenovirus vector (Ad26) is ranked no. 1557 at 102 ppm.

Figure 4 shows the relative expression of the three recombinant proteins from a HIV viral vector vaccine composition ("Env", "Mos1GagPol" and "Mos2GagPol").

Figure 5 shows the normalised relative expression of the three recombinant proteins from the data in Figure 4. The expression is normalised to MoslGagPol expression.

Figure 6 shows the recombinant protein from an Ebola virus vector vaccine composition ("EboTransgene") in the context of the A549 proteome. EboTrasgene is ranked 247 at 1027 ppm.

Figure 7 compares the protein expression levels of the Ebola virus recombinant protein by different batches of the virus vector vaccine composition.

Figure 8 shows the expression of HIV transgenes Mos1.Env (left graph) as analyzed using peptide IEPLGVAPTK (SEQ ID NO:25) based on SEQ ID NO:1, and Mos2S.Env (right graph) as analyzed using peptide TTLF[C]ASDAK (SEQ ID NO:27) based on SEQ ID NO:26 ([C] means cysteine was carbamidomethylated after synthesis).

Figure 9 shows the expression of Mos1.Env transgene (SEQ ID NO:1) as a function of cell seeding density (see legend, in number of cells per well in a 24-well cell plate) and viral load (expressed in MOI as based on the cell density 72h post seeding of 1.5e4 cells per well). MOI is expressed in number of viral particles per cell.

**EXAMPLES**

***Example 1: Simultaneous Analysis of Recombinant Protein Expression from a Trivalent Adenovirus Based HIV vaccine by Mass Spectrometry.***

The HIV vaccine drug product (Ad26.HIV DP) consists of a blend of three different Ad26 adenoviruses each encoding a different transgene designed to be expressed in human cells upon administration and subsequently induces an immune response. In this example a blend of Ad26.Env, Ad26.Mos1GagPol and Ad26.Mos2GagPol were present in the final Ad26.HIV DP in a ratio of 2:1:1, based on the amount of virus particles. The sequences of the recombinant proteins expressed from these transgenes are provided in the sequence listing: Env (SEQ ID NO: 1), Mos1GagPol (SEQ ID NO: 2) and Mos2GagPol (SEQ ID NO: 3). Both MoslGagPol (SEQ ID NO: 2) and Mos2GagPol (SEQ ID NO: 3) contain a mosaic of epitopes based on the HIV Gag and Pol proteins, and Env (SEQ ID NO: 1) contains a mosaic of epitopes based on the HIV Env [5].
Obtaining specific antibodies for the recombinant proteins encoded by either the MoslGagPol or the Mos2GagPol transgenes (*i.e.* antibodies recognising one protein but not the other) proved very difficult due to their high similarity. These two recombinant proteins are 89% identical in sequence and the differences are spread throughout the entire sequence as single amino acid changes. These sequence differences mean that distinctive epitopes are unlikely to be present for raising antibodies that are specific and selective for each protein, and hence no antibody to distinguish the two recombinant proteins could be obtained.

*Sample preparation*

A549 cells (adenocarcinomic human alveolar basal epithelial cells) grown to a density of $4.5 \times 10^6$ were infected with Ad26.HIV DP at an MOI of 50,000. The cells were harvested at 48h post infection. The experiment was performed in duplicate. The adherent cells were washed 3 times with ice cold PBS and lysed in 8M urea in 50mM ammonium bicarbonate and Roche complete mini protease inhibitor cocktail. The lysate was sonicated and centrifuged. The supernatant was collected and the clarified cell lysate (250 $\mu$g of total protein) was subjected to in-solution digestion with Lys-C (1:100 w/w) at 37°C for 4h, followed by a 4x-dilution to 2M urea and addition of trypsin (1:100 w/w). The resulting peptide mixture was desalted on a C18 Seppak column and dried *in vacuo.*

*Setup of the LC-MS/MS*

An Ultimate3000 nanoLC (Thermo Scientific), equipped with a Nano Trap pre-Column filled with Acclaim PepMap100 C18 (3 $\mu$m, 100Å; Thermo Scientific) and an EASY-Spray Column (PepMap RSLC, C18, 2$\mu$m, 100Å, 75$\mu$m x 50cm), was directly coupled to a qExactive Orbitrap mass spectrometer (Thermo Scientific). 500 ng of each full cell digest mentioned above was loaded on the pre-column using 100% buffer A (0.1% formic acid) at a flow rate of 5 $\mu$L/min for 10 min. Subsequently, peptides were eluted from the pre-column onto the analytical EASY-Spray column using the following gradient setup on the nanoLC operating at 200 nL/min: 0-15min 1% buffer (80% acetonitrile, 0.1% FA), between 15-120 min a gradient from 8-19.2% buffer B. 120-165min 19.2-40% buffer B; 165-168 min 40-80% buffer B, 168-169 at 80% buffer B, 169-170min 80-1% buffer B, and 170-185 min at 1% buffer B. This was adapted from Reference 76. Each digest was analysed on the LC-MS/MS setup in duplicate.

*Bioinformatics*

This resulted in 8 datasets which were analysed for recombinant protein expression. In each dataset, the MS data was used to identify the proteins present in the sample by a database search algorithm (*e.g.* Sequest [7]). Typical q-Exactive search parameters were used in terms of accuracy in MS and MS/MS mode to search the data against the entire human proteome, supplemented with the adenovirus proteins and the sequences of the three different recombinant proteins. False discovery rates were also estimated by a suitable algorithm (*e.g.* Percolator [9]). The data was filtered to a suitable level of false positives, here 1% at the identified MS/MS spectrum level.

To obtain expression levels of each protein present in the dataset, MS data characteristics were converted to represent semi-absolute expression levels. The ratio of identified MS/MS spectra for a protein over its molecular weight was calculated. These ratios were subsequently normalised to the sum of all ratios in the dataset and expressed in ppm.

*Results*

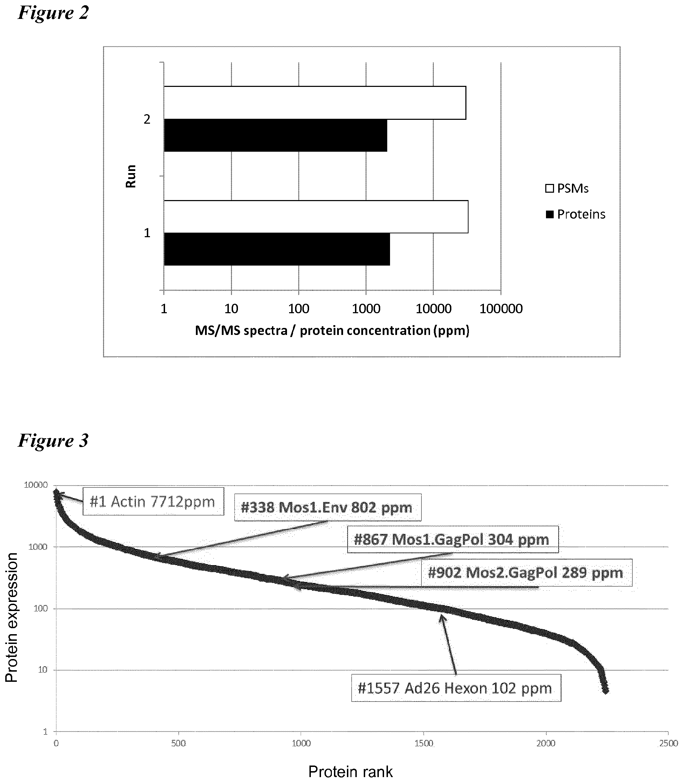

The results from the duplicate LC-MS/MS-run of each sample were combined to increase accuracy on the MS-based quantitation. This resulted in the two datasets presented in Figure 2 (Runs 1 and 2). These two datasets proved highly comparable in terms of the amount of proteins and MS/MS spectra (PSMs) that were identified. The observed small variation is typical for the approach and differences were seen close to the detection limit of the method.

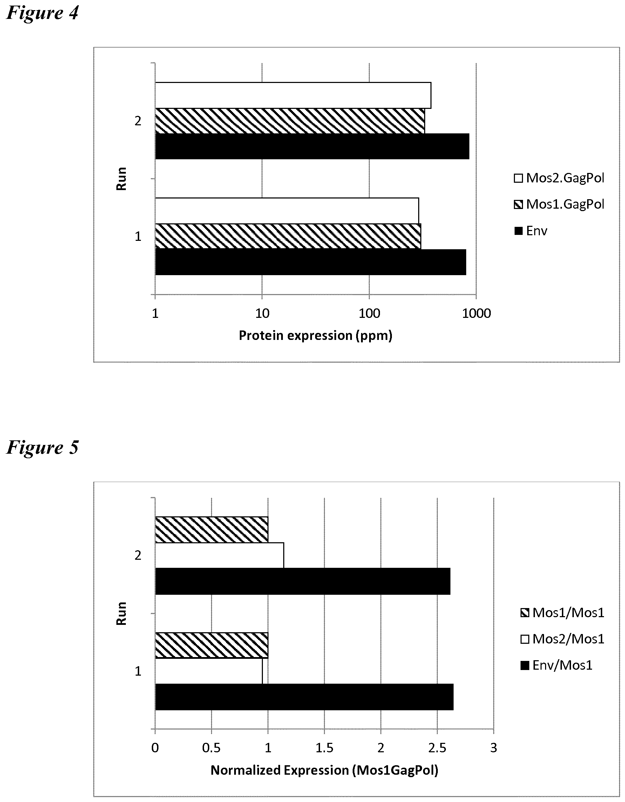

The three recombinant proteins were readily observed in each run (Figures 3 and 4) and the normalised expression ratios of the transgenes were in close agreement between the 2 experiments (normalization to Ad26.Mos1GagPol expression, Figure 5). Normalizing the expression ratios revealed that recombinant protein expression of the Env, Mos1GagPol and Mos2GagPol were in close agreement with the blending ratio in the Ad26.HIV DP which was 2:1:1.

Furthermore, the level of a protein from the adenovirus vector, *e.g.* Hexon (see Figure 3), especially when compared with the level of a cellular protein, provides an indication of the infection efficiency of the adenovirus vector. Besides providing a relative expression ratio, the data presented above could also be mined for proteotypic peptides of each recombinant protein. These can be developed into a more accurate quantitative assay based on single reaction monitoring (SRM).

***Example 2: Batch to Batch comparison of Potency by Mass Spectrometry Based Recombinant Transgene Expression Analysis***

The potency of several batches of a monovalent Ebola (Ad26.ZEBOV) vaccine encoding a single recombinant protein from an Ebola strain in an adenovirus 26 virus was tested.

*Methods*

A549 cells were infected with 5 different batches of the Ad26.ZEBOV vaccine at an MOI of 25,000, in triplicate. Cells were harvested 48h post-infection and lysed according to the methodology described in Example 1. Sample work-up towards the q-Exactive Orbitrap was executed identical to Example 1. The peptide mixtures were analysed using the same protocols as in Example 1. The Orbitrap LC-MS/MS data was searched against the human database, supplemented with three versions of the recombinant protein from the different Ebola strains.

*Results*

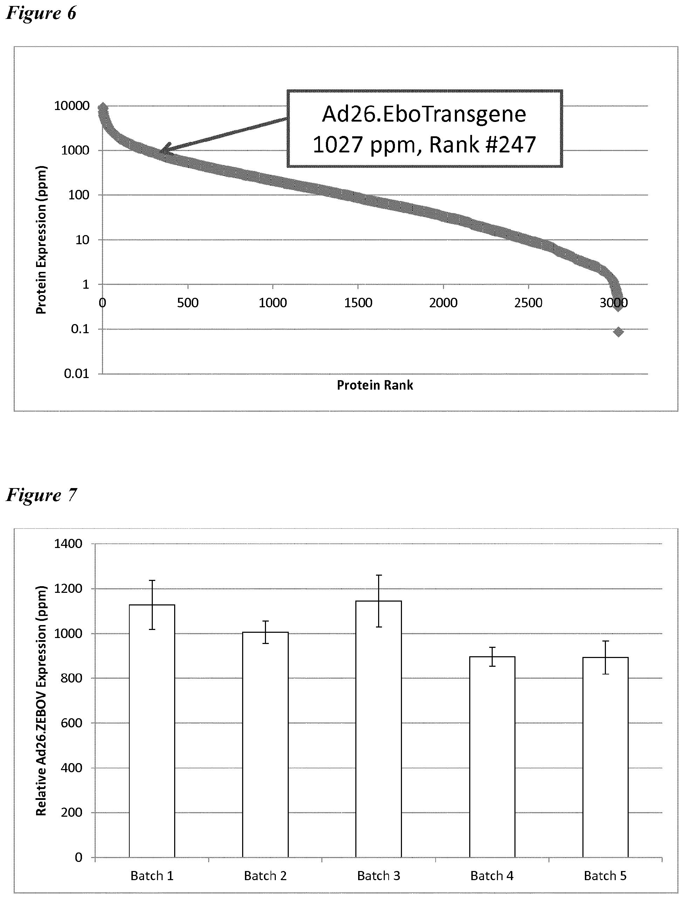

After database searching, the Ebola recombinant protein encoded by the Ad26 virus could readily be identified by several unique peptide sequences, without interfering identifications of the other, similar Ebola proteins, indicating the specificity of the approach. Subsequently, spectral count based relative quantitation was used to estimate the expression level of the Ebola transgene in the context of the cellular lysate (Figures 6 and 7). On average, all combined data indicated an expression level of 1027 ppm and ranked the Ebola recombinant protein among the 300th most abundantly expressed proteins in the A549 cell lysate (Rank #247).

When inspecting the five triplicate experiments separately, small variations within triplicates were observed (*i.e.* relative standard deviations (RSD) of 4.9-10.2%). These variations reflect variation occurring throughout sample preparation and LC-MS/MS analysis.

***Example 3: Identifying Proteotypic Peptides useful for Absolute Quantitation of Proteins by Mass Spectrometry***

The aim of this example was to identify proteotypic peptides that are useful for synthesis of stable isotope labelled analogues as internal references for absolute quantitation of the proteins by MS. Certain peptides have been selected from the MS analysis in Example 1 to be useful proteotypic peptides in consistently identifying the proteins of interest. Tables 1 and 2 show the characteristics of these proteotypic peptides in multiple reaction monitoring (MRM)-transitions for the negative ion mode and the positive ion mode, respectively. The average molecular weight of the peptide (MW), the precursor mass in the first quadrupole ($Q_1$; m/z), the predicted fragment mass in the third quadrupole ($Q_3$; m/z) are depicted. To optimize the fragmentation behaviour of each peptide in the the quadrupole ion trap to be able to monitor the best transition(s) (Q1-Q3 combinations) with the highest specificity and sensitivity, several parameters were optimized using synthetic versions of each proteotypic peptide identified in example 1 above. The retention time (Rt; min), the differential pulse voltammetry (DPV), Collission Energy (CE) and CXP were monitored/optimized in both negative (Table 1) and positive (Table 2) ion mode, respectively.

Table 1: Characteristics of proteotypic peptides in MRM-transitions for the negative ion mode.

| Peptide Name | Amino acid sequence | Average MW | Q1 Mass (m/z) | Q3 Mass (m/z) | Rt (min) | DPV | CE | CXP |
|---|---|---|---|---|---|---|---|---|
| **Transgene peptides** | | | | | | | | |
| Env4 | AIEAQQHLLQLTVWGIK (SEQ ID NO: 4) | 1948.3 | 973.0 | 950.7 | 4.3 | -66 | -48 | -29 |
| Mos1.3 | IGPENPYNTPVFAIK (SEQ ID NO: 5) | 1659.9 | 828.8 | 806.9 | 3.7 | -63 | -36 | -25 |
| Mos2.1 | IGPENPYNTPIFAIK (SEQ ID NO: 6) | 1673.9 | 835.8 | 814 | 3.9 | -40 | -38 | -27 |
| Mos2.2 | YTAFTIPSINNETPGIR (SEQ ID NO: 7) | 1894.1 | 945.8 | 923.9 | 3.9 | -105 | -36 | -27 |
| **Housekeeping peptides** | | | | | | | | |
| Act1 | SYELPDGQVITIGNER (SEQ ID NO: 8) | 1790.9 | 894.3 | 885.2 | 3.5 | -95 | -38 | -27 |
| G3P1 | LISWYDNEFGYSNR (SEQ ID NO: 9) | 1763.9 | 880.7 | 871.8 | 3.7 | -90 | -36 | -29 |
| G3P3a | AVGKVIPEELNGK (SEQ ID NO: 10) | 1353.6 | 675.8 | 666.9 | 2.5 | -90 | -30 | -19 |
| **Viral peptide** | | | | | | | | |
| VII1 | TTVDDVIDSVVADAR (SEQ ID NO: 11) | 1575.7 | 786.7 | 777.5 | 4.2 | -86 | -32 | -25 |

Table 2: Characteristics of proteotypic peptides in MRM-transitions for the positive ion mode.

| Peptide Name | Amino acid sequence | Average MW | Q1 Mass (m/z) | Q3 Mass (m/z) | Rt (min) | DP V | CE | CXP |
|---|---|---|---|---|---|---|---|---|
| **Transgene peptides** | | | | | | | | |
| Env1 | EATTTLFZASDAK (SEQ ID NO: 12) | 1414.5 | 708.0 | 798.6 | 2.7 | 70 | 31 | 23 |
| Env2 | VSFEPIPIHYZAPAGFAILK (SEQ ID NO: 13) | 2230.6 | 744.4 | 884.7 | 4.5 | 75 | 31 | 30 |
| Env3 | AFYTAGDIIGDIR (SEQ ID NO: 14) | 1411.6 | 706.6 | 858.7 | 3.9 | 75 | 29 | 30 |
| Env3a | AFYTAGDIIGDIR (SEQ ID NO: 14) | 1411.6 | 706.6 | 1030.7 | 3.9 | 75 | 33 | 36 |
| Mos1.1 | FAVNPGLLETSEGZR (SEQ ID NO: 15) | 1649.8 | 825.7 | 1218.7 | 3.4 | 56 | 37 | 38 |
| Mos1.2 | SLYNTVATLYZVHQR (SEQ ID NO: 16) | 1825.1 | 913.3 | 1147.5 | 3.9 | 65 | 49 | 28 |
| Mos1.4 | IVSLTETTNQK (SEQ ID NO: 17) | 1233.4 | 617.5 | 1021. 7 | 2.3 | 54 | 27 | 32 |
| Mos2.3 | IATESIVIWGK (SEQ ID NO: 18) | 1216.4 | 609.0 | 600 | 3.6 | 61 | 25 | 20 |
| Mos2.3a | IATESIVIWGK (SEQ ID NO: 18) | 1216.4 | 609.0 | 1032.8 | 3.6 | 61 | 27 | 34 |
| Mos2.4 | VVSLTDTTNQK (SEQ ID NO: 19) | 1205.3 | 603.5 | 1007.7 | 2.1 | 66 | 27 | 32 |
| Mos2.5 | FALNPGLLETSEGZK (SEQ ID NO: 20) | 1635.8 | 818.7 | 1190.8 | 3.7 | 50 | 37 | 42 |
| **Housekeeping peptides** | | | | | | | | |
| G3P2 | VIPELNGK (SEQ ID NO: 21) | 869 | 435.5 | 657.3 | 2.4 | 43 | 19 | 20 |
| G3P3a | AVGKVIPEELNGK (SEQ ID NO: 22) | 1353.6 | 452.1 | 592.6 | 2.5 | 50 | 19 | 20 |
| **Viral peptides** | | | | | | | | |
| Hex1 | ATDTYFSLGNK (SEQ ID NO: 23) | 1216.3 | 608.9 | 665.5 | 2.9 | 66 | 29 | 22 |
| IX1 | LLALLAELEALSR (SEQ ID NO: 24) | 1411.7 | 706.6 | 888.7 | 5.8 | 90 | 31 | 28 |

*Preparation of calibration samples*

Synthetic peptides from Tables 1 and 2 were obtained from JPT Peptide Technologies (Berlin, Germany). The peptides were dissolved in 10% formic acid to obtain stock concentrations of 0.1 mg/mL. Combined working solution in 10% formic acid were prepared for all peptides with an estimated starting concentration of 1000 ng/mL. Calibration curves ranging from 0.1-1000 ng/mL were prepared.

*Sample preparation and MS analysis*

Using the SRM parameters determined for each proteotypic peptide above, a first estimation of transgene expression can be performed. A549 cells were transfected with samples by different batches of the HIV vaccine drug product Ad26.HIV DP (see Example 1; Samples 1 and 2), separately, as described in Example 1. A549 cells transfected with an empty adenovirus vector (*i.e.* homologues adenovirus vectors that do not encode any recombinant proteins), Ad26.DE3.5ORF6 using the same protocol (Control 1) and an uninfected A549 cell extract (Control 2) were used as negative controls. Adenovirus vectors encoding Env only, MoslGagPol only and Mos2GagPol only were also used as controls (Controls 3-5, respectively) in order to demonstrate that the method could quantify specifically and precisely highly similar recombinant proteins.

The cells were harvested and the cell lysates were prepared for MS analysis as described in Example 1. The samples were diluted by 1/2 and 1/10 in 10% formic acid.

*MS analysis*

The samples were injected into a triple quadrupole mass spectrometer API-6500 (AB Sciex), and the data were analysed using the Turbo-Ionspray™ Interface (AB Sciex) in negative and positive-ion modes. The SRM settings depicted in Table 1 were used.

*Results*

The amounts of the proteotypic peptides for the recombinant proteins (ENV, MoslGagPol and Mos2GagPol), of the cellular proteins and of the viral vector proteins (the capsid proteins of the viral particle) were determined, and the results are shown in Tables 3-7.

Table 3: Results for the proteotypic peptides for the transgene ENV. (ND = not detected; results in Italics are above quantitation limit and need further optimization of the transitions to be used; (+) = positive ion mode was used; (-) = negative ion mode was used)

| Sample | ENV1(+) | ENV2(+) | ENV3(+) | ENV4(-) |
|---|---|---|---|---|
| **1/10 diluted samples** | nM | | | |
| Control 1 | ND | ND | ND | ND |
| Control 2 | ND | ND | ND | ND |
| Control 3 | 242 | 2815 | 672 | 402 |
| Control 4 | ND | ND | ND | ND |
| Control 5 | ND | ND | ND | ND |
| Sample 1 | 83 | 995 | 341 | 227 |
| Sample 2 | 83 | *883* | 343 | 267 |
| **1/2 diluted samples** | nM | | | |
| Control 1 | ND | ND | ND | ND |
| Control 2 | ND | ND | ND | ND |
| Control 3 | *256* | *2421* | *384* | *215* |
| Control 4 | ND | ND | traces | ND |
| Control 5 | ND | ND | ND | ND |
| Sample 1 | 91 | *893* | 227 | *136* |
| Sample 2 | 79 | *753* | 191 | *147* |

Table 4: Results for 4 proteotypic peptides for the transgene Mos1GagPol. (ND = not detected; BQL = below quantitation limit; (+) = positive ion mode was used; (-) = negative ion mode was used)

| Sample | Mos1.1(+) | Mos1.2(+) | Mos1.3(-) | Mos1.4(+) |
|---|---|---|---|---|
| **1/10 diluted samples** | nM | | | |
| Control 1 | ND | ND | ND | ND |
| Control 2 | ND | ND | ND | ND |
| Control 3 | ND | ND | ND | ND |
| Control 4 | 184 | 274 | 57 | 30 |
| Control 5 | ND | ND | ND | ND |
| Sample 1 | 21 | BQL | 8 | 2 |
| Sample 2 | 21 | BQL | 8 | 2 |

(continued)

| 1/2 diluted samples | nM | | | |
|---|---|---|---|---|
| Control 1 | ND | ND | ND | ND |
| Control 2 | ND | ND | ND | ND |
| Control 3 | ND | ND | ND | ND |
| Control 4 | 160 | 238 | 34 | 45 |
| Control 5 | ND | ND | ND | ND |
| Sample 1 | 18 | 36 | 5 | 2 |
| Sample 2 | 17 | 24 | 5 | 2 |

Table 5: Results for 5 proteotypic peptides for the transgene Mos2GagPol. (ND = not detected; BQL = below quantitation limit; (+) = positive ion mode was used; (-) = negative ion mode was used)

| Sample | Mos2.1 (-) | Mos2.2 (-) | Mos2.3 (+) | Mos2.4 (+) | Mos2.5 (+) |
|---|---|---|---|---|---|
| 1/10 diluted samples | | nM | | | |
| Control 1 | ND | ND | ND | ND | ND |
| Control 2 | ND | ND | ND | ND | ND |
| Control 3 | ND | ND | ND | ND | ND |
| Control 4 | ND | ND | ND | ND | ND |
| Control 5 | 131 | 458 | 86 | 38 | 147 |
| Sample 1 | 19 | BQL | BQL | 1 | BQL |
| Sample 2 | 16 | BQL | BQL | 2 | BQL |
| 1/2 diluted samples | | nM | | | |
| Control 1 | ND | ND | ND | ND | ND |
| Control 2 | ND | ND | ND | ND | ND |
| Control 3 | ND | ND | ND | ND | ND |
| Control 4 | ND | ND | ND | ND | ND |
| Control 5 | 70 | 211 | 68 | 34 | 105 |
| Sample 1 | 11 | 58 | 6 | 1 | 8 |
| Sample 2 | 8 | BQL | 5 | 1 | 8 |

Table 6: Results for proteotypic peptides for the cellular proteins: Actin and G3P. (ND = not detected; results in Italics are above quantitation limit; (+) = positive ion mode was used; (-) = negative ion mode was used)

| Sample | Act (-) | G3P1 (-) | G3P2 (+) |
|---|---|---|---|
| 1/10 diluted samples | | nM | |
| Control 1 | *3392* | 913 | 162 |
| Control 2 | *9075* | 2041 | 672 |
| Control 3 | *6817* | 1621 | 452 |
| Control 4 | *3084* | 839 | 131 |

(continued)

| Sample | Act (-) | G3P1 (-) | G3P2 (+) |
|---|---|---|---|
| **1/10 diluted samples** | nM | | |
| Control 5 | *8007* | 1996 | 598 |
| Sample 1 | *5066* | 1327 | 252 |
| Sample 2 | *4196* | 1191 | 211 |
| **1/2 diluted samples** | nM | | |
| Control 1 | *2269* | 635 | 174 |
| Control 2 | *4692* | 1118 | 550 |
| Control 3 | *4141* | 1020 | 437 |
| Control 4 | *2132* | 607 | 153 |
| Control 5 | *4383* | 1191 | 513 |
| Sample 1 | *3304* | 934 | 276 |
| Sample 2 | *2511* | 717 | 195 |

Table 7: Results for proteotypic peptides for proteins of the viral vector: Hex, VII and IX1. (ND = not detected; results in Italics are above quantitation limit; (+) = positive ion mode was used; (-) = negative ion mode was used)

| Sample | Hex (+) | IX1 (+) | VII (-) |
|---|---|---|---|
| **1/10 diluted samples** | nM | | |
| Control 1 | 13.4 | *6305* | 307 |
| Control 2 | 52.5 | *20259* | 717 |
| Control 3 | 7.0 | *1665* | 265 |
| Control 4 | 3.5 | 273 | 154 |
| Control 5 | 9.3 | 275 | 291 |
| Sample 1 | 4.0 | BQL | 194 |
| Sample 2 | 3.5 | BQL | 204 |
| **1/2 diluted samples** | nM | | |
| Control 1 | 15.8 | *7027* | 164 |
| Control 2 | 41.9 | *14167* | 307 |
| Control 3 | 7.4 | *1238* | 155 |
| Control 4 | 3.8 | 252 | 99 |
| Control 5 | 8.6 | 179 | 132 |
| Sample 1 | 4.4 | 58 | 103 |
| Sample 2 | 3.2 | 56 | 101 |

*Discussion and conclusions*

This example demonstrates that the transitions of many selected proteotypic peptides were highly specific for identifying, and quantifying, the proteins of interest. In particular, each specific recombinant protein was detected in the transfected cells, while no recombinant proteins were detected in the viral empty vector construct transfected cells. Controls 3-5 across Tables 3-5 show specific detection of the relevant recombinant proteins based on their proteotypic peptides when individually expressed. The viral proteins were detected in all transfected cells.

The amounts determined for the diluted samples (1/2 and 1/10) showed a good correlation for most peptides analysed in the positive ion, whereas currently much less correlation was observed for some of the peptides analysed in the negative ion mode.

The cellular proteins were detected at high concentration levels in all samples, but the concentration levels varied by a factor of 3 between the samples. This could be attributed to the inaccuracy of the calibration curve because no detailed information on the purity or the solubility of the synthetic peptides was provided. It was also noted that variation in the samples may arise because neither the peptidase digestion efficiency nor the stability of the individual peptides during the digestion process have been evaluated at this stage. These parameters can be controlled and optimised in future experiments.

For a number of peptides, the calibration range was sub-optimal, so the readings were either above the quantitation limit or below the quantitation limit. For example, some samples showed concentration levels above the upper limit of quantification at the 1/10 dilution (Env2, Act, G3P, IX1). To improve the readings, the calibration range can be extended in future experiments, as well as the optimal dilution at which to measure these peptides.

It was also noted that, since the calibration samples were prepared synthetically, the matrix of the calibration samples was different from the matrix of the study samples. To evaluate potential matrix effects, a peptidase digest of a blank cell lysate (*i.e.* the cell was not infected with any viral vectors) was used (Control 2). The accuracy was acceptable for most peptides.

From the results of this example, the following proteotypic peptides are considered to be useful for the synthesis of stable isotope labelled analogues for absolute quantitation of the proteins by MS analysis (as explained in Stage 2 of the MS analysis above):

- Env: Env1 and Env2.
- Mos1GagPol: Mos1.4, and Mos1.2 or Mos1.3.
- Mos2GagPol: Mos2.4, and Mos2.5 or Mos2.3.
- Viral vector protein: Hex1.
- Cellular protein: Act and G3P2.

Thus, the proteotypic peptides for Env, MoslGagPol and Mos2GagPol can provide an absolute quantitation of the level of each of the recombinant proteins in the cell lysate. This provides information on how effectively the vaccine composition causes expression of the recombinant proteins encoded by its viral vector components, and hence the *in vitro* potency of the vaccine composition.

The proteotypic peptide for the viral vector protein can provide an absolute quantitation of the level of the protein in the cell lysate. This provides information on the infectivity of the adenovirus vector of the vaccine composition.

### *Example 4: Proteotypic Peptides for Absolute Quantitation of Env proteins*

Determination of transgene expression is achieved by the methodology described in example 3.

In this example, the level of two HIV vaccine transgenes expressed by two different adenovirus vectors was determined in A549 cells in which the adenovirus vectors cannot replicate. The A549 cells were seeded on 24 well cell growing plates and left to grow to confluency for 72h. Cells were then infected with the HIV drug product blend consisting of the two different adenovirus vectors, each containing a different HIV transgene based on the envelope proteins; Mos1.Env (SEQ ID NO:1) and Mos2S.Env (SEQ ID NO:26). The blending ratio was 1:1 based on virus particles per mL. Each transgene was represented by a single proteotypic peptide (SEQ ID NO:25 for Mos1.Env, and SEQ ID NO:27 for Mos2S.Env).

Different multiplicities of infection (MOI) were used, ranging from 70,000 down to 3250 virus particles per cell. Each MOI was performed in triplicate. 48h post infection, the cells were washed three times with ice cold PBS. Cells were then lysed in 8M urea. The lysate was transferred to a 96 well plate which was sonicated to improve solubility of each protein in the lysate. After reduction in DTT and alkylation using iodoacetamide, samples were digested in 96 well plate format using LysC (4h) and trypsin (18h) sequentially. The resulting peptide mixtures were spiked with the heavy labelled internal standard proteotypic peptides and subsequently desalted, dried *in vacuo* and stored at -20°C until LC-MRM-MS analysis on an AB-Sciex Q-Trap 6500.

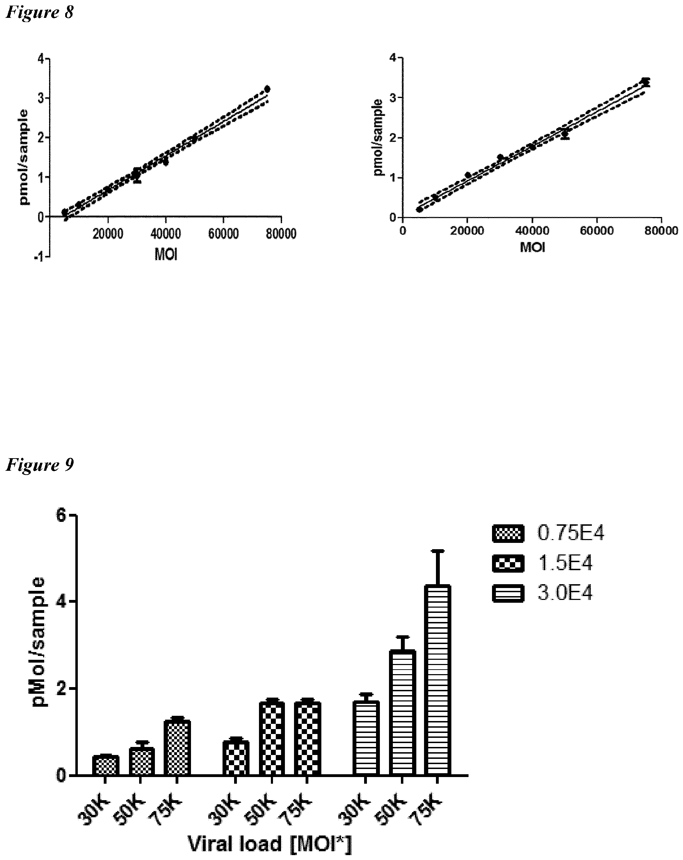

Each peptide was quantified using one transition (Table 2). Run to run equilibration was performed using the spiked heavy labelled proteotypic peptide. After equilibration, the area under the curve of the light peptide transition was quantified using a standard curve of a synthesized version of the light peptide. The results for both Mos1.Env and Mos2S.Env transgene expression were analyzed from the same MS runs and the expression levels (in pmol/well of cells) are depicted in Figure 8. It can be concluded that both transgenes (Fig.8 left graph for Mos1.Env and Fig.8 right graph for Mos2S.Env) show merely equal expression levels for each MOI. Also, the correlation between MOI and expression is linear within the range tested. The error bars represent the triplicates at cell infection level. This shows that the expression of several transgenes in a single vaccine composition can be determined in a broad range with high selectivity and precision.

### Example 5: Optimizing cell density and MOI for an adenoviral vector vaccine component

In this example, the methodology described in example 3 and 4 is amended to further investigate the impact of cell density on the 24-well plate and the multiplicity of infection of the adenovirus viral vaccine composition comprising an adenoviral vector vaccine component carrying the HIV transgene Mos1.Env (SEQ ID NO:1). In this experiment, cells were seeded at three different densities, i.e. 7500/well, 15,000/well or 30,000 per well, in a 24 well plate. After seeding, cells were left to grow for 72h and were then infected with three different MOIs, i.e. 30,000 viral particles (VP)/cell, 50,000 VP/cell or 75,000 VP/cell (as based on the cell count achieved in the 15,000 cells/well after 72h). In other words, cells were infected with an equal amount of virus particles/well, regardless of cell density. Similar to example 4, these experiments were also performed in triplicate at the level of cell infection. After following the same sample preparation as described in example 4, the spiked peptide mixtures from each well were analyzed using the same MS setup as used in example 4. The results are depicted in Figure 9. The expression levels are expressed in pmol transgene per well (sample). These data indicate that cell density has a distinct impact on transgene expression level. From these experiments we concluded that higher cell densities give higher expression when exposed to identical numbers of virus particles in the well. Such experiments are useful in setting up the optimal cell seeding and infection conditions for a relative potency experiment in which a test article is compared to a reference batch.

It will be understood that the invention has been described by way of example only.

### REFERENCES

[1] Have et al., 2012, Biologicals, 40(1):84-7.
[2] Rezapkin et al., 2005, Biologicals, 33(1):17-27.
[3] Badger et al., Vaccine, 2011, 29(39):6728-35.
[4] Weiner et al., Molecular Therapy, 21, 506-508
[5] Barouch et al., 2010, Nat Med 16(3): 319-323.
[6] Hu et al., J Mass Spectrom. 2005 Apr;40(4):430-43.
[7] Eng et al., 1994, J Am Soc Mass Spectrom 5(11): 976-989.
[8] Perkins et al., 1999, Electrophoresis 20 (18): 3551-67
[9] Käll et al., 2007, Nature Methods 4:923 - 925.
[10] Peng et al., Nat Methods. 2012, 9(6):524-5.
[11] Schwanhäusser et al., 2011, Nature 473(7347):337-42. Erratum in: Nature. 2013 Mar 7;495(7439): 126-7.
[12] Silva et al., 2005, Anal Chem, 77(7):2187-200.
[13] Aye et al., 2010, Mol Biosyst, 6(10):1917-27.
[14] Malmström et al., 2009, Nature 460(7256):762-5.
[15] Silva et al. 2006, Mol Cell Proteomics 5:144-156.
[16] Grossmann et al., 2010, J Proteomics 73:1740-1746.
[17] Kondrat et al., 1978, Anal Chem 50(14):2017-21.
[18] Gergov et al., J. Chromatogr., B: Anal. Technol. Biomed. Life. Sci. 2003, 795(1): 41-53.
[19] Kuzyk et al., 2009, Mol. Cell. Proteomics 8(8):1860-1877.
[20] Seppala et al., 2011, J. proteome, 10, 2113-2122.
[21] Kiyonami et al., 2011, Mol.Cell. Proteomics 10 (2), No. M110.002931.
[22] Gerber et al., 2003, PNAS, 100(12):6940-45.
[23] Brun et al., 2009, J. Proteomics, 72(5):740-49.
[24] Shiver et al., 2002, Nature 415(6869): 331-5.
[25] Hill et al., Hum Vaccine 6(1): 78-83.
[26] Sullivan et al., 2000, Nature 408(6812): 605-9.
[27] Santra et al., 2009, Vaccine 27(42): 5837-45.
[28] WO 2012/082918.
[29] U.S. Pat. No. 5,385,839.
[30] U.S. Pat. No. 5,559,099.
[31] U.S. Pat. No. 5,837,511.
[32] U.S. Pat. No. 5,846,782.
[33] U.S. Pat. No. 5,851,806.

[34] Thomas Shenk, "Adenoviridae and their Replication," M. S. Horwitz, "Adenoviruses," Chapters 67 and 68, respectively, in Virology, B. N. Fields et al., eds., 3d ed., Raven Press, Ltd., New York (1996)

[35] U.S. Pat. No. 5,994,106.

[36] U.S. Pat. No. 5,994,128.

[37] U.S. Pat. No. 5,965,541.

[38] U.S. Pat. No. 5,981,225.

[39] U.S. Pat. No. 6,040,174.

[40] U.S. Pat. No. 6,020,191.

[41] U.S. Pat. No. 6,113,913.

[42] Sambrook et al., Molecular Cloning, a Laboratory Manual, 2d ed., Cold Spring Harbor Press, Cold Spring Harbor, N.Y. (1989).

[43] Watson et al., Recombinant DNA, 2d ed., Scientific American Books (1992),

[44] Ausubel et al., Current Protocols in Molecular Biology, Wiley Interscience Publishers, NY (1995)

[45] Wang et al., 2004. Virology 330: 375-83

[46] U.S. Patent No. 5,494,807

[47] U.S. Pat. No. 5, 185, 146

[48] Mackett et al., 1982, Proc. Natl. Acad. Sci. USA 79:741 5-7419.

[49] U.S. Patent No. 5,093,258.

[50] U.S. Pat. No. 5,445,953.

[51] EP 0 308 220 A1.

[52] Medina et al., 2001, Vaccine 19(13-14):1573-1580.

[53] Garmory et al., 2003, J Drug Target 11(8-10):471–479.

[54] Weiss and Krusch, 2001, Biol. Chem. 382: 533-41.

[55] Darji et al. 2000, FEMS Immunol and Medical Microbiology 27: 341-9

[56] U.S. Pat. No. 5,830,463.

[57] Stubbs et al. 2001, Nature Medicine 7: 625-29.

[58] Sullivan et al., 2003, Nature 424(6949):681-684.

[59] Sullivan, et al., 2006, PLoS Med 3(6): e177.

[60] Geisbert et al., 2011, J Virol 85: 4222-4233.

[61] WO 2009/026183.

[62] WO 2010/096561.

[63] WO 2006/120034.

[64] WO 02/22080.

[65] WO 01/02607.

[66] Zhou et al., 2010, Mol Ther 18:2182-9.

[67] Hu et al., 2011, Virus Res 155: 156-62;

[68] Vemula and Mittal, 2010, Expert Opin Biol Ther 10: 1469-87.

[69] WO 2004/037294.

[70] Zhou et al., 2006, Mol Ther 14: 662-672.

[71] Yang and Zubarev, 2011, Electrophoresis 31: 1764-1772.

[72] Diepold et al., 2012, PLoS One, 7:e30295.

[73] JP-A-3-22979

[74] JP-A-2-227075

[75] WO 97/10354

[76] Corradini et al., 2014, Mol Cell Proteomics, 13: 2004-2016.

[77] Williams T L et al. : "Simultaneous quantification of hemagglutinin and neuramidase of influenza virus using isoptope dilution mass spectroscopy", Vaccine, Elsevier LTD, GB, vol. 30. no. 14, 23 March 2012, pages 2475-2482.

[78] Melkamu Getie-Kebtie et al. : "Label-free mass spectrometry-based quantification of hemagglutinin and neuraminidase in influenza virus preparation and vaccines". Influenza and other respiratory viruses, vol. 7, no.4, 3 July 2013, pages 521-530

[79] Wanda I. Santana et al. : Quantification of Viral Proteins of the Avian H7 subtype of Influenza Virus: An Isotope Dilution Mass Spectrometry Method Applicable for Producing more Rapid Vaccines in the case of an Influenza Pandemic", Analytical Chemistry, vol. 86, no. 9, 1 April 2014, pages 4088-4095.

[80] WO 2014/134561

[81] EP 1118860

## Claims

1. A method of potency testing a viral vector vaccine composition encoding a recombinant protein or a nucleic acid vaccine composition encoding a recombinant protein, wherein the method comprises:

   (i) infecting a cell culture with the viral vector vaccine composition or nucleic acid vaccine composition;
   (ii) lysing said cell culture to form a cell lysate; and
   (iii) quantifying the recombinant protein encoded by the viral vector vaccine composition or the nucleic acid vaccine composition as expressed in the cell culture using mass spectrometry; and wherein the method does not require any additional steps to purify the recombinant protein from other proteins in the cell lysate

2. The method according to claim 1, wherein the viral vector vaccine composition comprises an adenovirus vector vaccine component encoding the recombinant protein, such as wherein the viral vector vaccine composition encodes at least two recombinant proteins.

3. The method according to claim 2, wherein the viral vector vaccine composition comprises (i) an adenovirus vector vaccine component encoding at least two recombinant proteins and/or (ii) multiple different adenovirus vector vaccine components encoding different recombinant proteins.

4. The method according to claim 1, wherein the viral vector vaccine composition comprises a poxvirus vector vaccine component encoding the recombinant protein.

5. The method according to any preceding claim, wherein:

   (i) two or more recombinant proteins are quantified using mass spectrometry;
   (ii) another protein in the cell culture has at least 85% identity to the recombinant protein, such as wherein the other protein is also encoded by the viral vector vaccine composition or the nucleic acid vaccine composition, or is a protein from the cells in the cell culture infected by the viral vector vaccine composition or the nucleic acid vaccine composition; and/or
   (iii) the quantifying is determining the relative or the absolute expression level of the recombinant protein.

6. The method of claim 5(iii), wherein the determining is:

   (i) determining the relative expression level of the recombinant protein comprising the steps of: (a) identifying proteins in a sample by mass spectrometry analysis; and (b) determining the relative expression level of each identified protein, including the recombinant protein, optionally wherein the relative determination is performed by determining expression of the recombinant protein relative to a cellular marker protein; or
   (ii) determining the absolute expression level of the recombinant protein comprising the steps of: (a) identifying proteins in a sample by mass spectrometry analysis; (b) determining the relative expression level of each identified protein, including the recombinant protein; (c) detecting a proteotypic peptide of the recombinant protein; and (d) determining the absolute expression level of the recombinant protein by comparing the detected amount of the proteotypic peptide to a known standard.

7. A method of determining the infectivity of a viral vector vaccine composition comprising:

   (i) infecting a cell culture with a viral vector vaccine composition;
   (ii) lysing said cell culture to form a cell lysate; and
   (iii) quantifying the intracellular level of a protein of a viral vector component of the viral vector vaccine composition in the cell culture using mass spectrometry; wherein the method does not require any additional steps to purify the recombinant protein from other proteins in the cell lysate.

8. The method according to claim 7, wherein the viral vector vaccine component is an adenovirus vector vaccine component.

9. The method according to claim 8, wherein the viral vector vaccine composition comprises (i) an adenovirus vector vaccine component encoding at least two recombinant proteins and/or (ii) multiple different adenovirus vector vaccine components encoding different recombinant proteins.

10. The method according to claim 7, wherein the viral vector vaccine component is a poxvirus vector vaccine component.

11. The method according to any one of claims 7-10, wherein:

   (i) two or more proteins are quantified using mass spectrometry;
   (ii) another protein in the cell culture has at least 85% identity to the protein of a viral vector vaccine component, such as wherein the other protein is a protein of another viral vector vaccine component of the viral vector vaccine composition, is encoded by the viral vector vaccine composition, or is a protein from the cells in the cell culture infected by the viral vector vaccine composition; and/or
   (iii) the quantifying is determining the relative or the absolute expression level of the protein of a viral vector vaccine component.

12. The method of claim 11(iii), wherein the determining is:

   (i) determining the relative expression level of the protein of a viral vector vaccine component comprising the steps of: (a) identifying proteins in a sample by mass spectrometry analysis; and (b) determining the relative expression level of each identified protein, including the protein of a viral vector vaccine component, optionally wherein the relative determination is performed by determining expression of the recombinant protein relative to a cellular marker protein; or

(ii) determining the absolute expression level of the protein of a viral vector vaccine component comprising the steps of: (a) identifying proteins in a sample by mass spectrometry analysis; (b) determining the relative expression level of each identified protein, including the protein of a viral vector vaccine component; (c) detecting a proteotypic peptide of the protein of a viral vector vaccine component; and (d) determining the absolute expression level of the protein of a viral vector vaccine component by comparing the detected amount of the proteotypic peptide to a known standard.

13. A method of testing the potency and the infectivity of a viral vector vaccine composition, comprising determining potency by a method according to any one of claims 1-6 and determining the infectivity by a method according to any one of claims 7-12, optionally in the same mass spectrometry analysis.

14. A method of manufacturing a viral vector vaccine composition encoding a recombinant protein or a nucleic acid vaccine composition encoding a recombinant protein, comprising potency testing the vaccine using a method according to any one of claims 1-6, determining the infectivity of a viral vector vaccine composition using a method according to any one of claims 7-12, or determining both the potency and infectivity of a viral vector vaccine composition using the method of claim 13.

15. A method of manufacturing vaccine doses of a viral vector vaccine composition encoding a recombinant protein or a nucleic acid vaccine composition encoding a recombinant protein, comprising steps of: (i) assaying the relative potency of the vaccine in a bulk of vaccine using a method according to any one of claims 1-6, determining the infectivity of a viral vector vaccine in a bulk of vaccine using a method according to any one of claims 7-12, or determining both potency and infectivity of a viral vector vaccine in a bulk of vaccine using the method of claim 13; and, if the results of step (i) indicate an acceptable relative potency and/or infectivity, (ii) dispensing the bulk vaccine into doses.

## Patentansprüche

1. Verfahren für die Wirksamkeitsprüfung einer Vektorimpfstoffzusammensetzung, die für ein rekombinantes Protein kodiert, oder einer nukleinsäurebasierten Impfstoffzusammensetzung, die für ein rekombinantes Protein kodiert, wobei das Verfahren umfasst:

(i) Infizieren einer Zellkultur mit der Vektorimpfstoffzusammensetzung oder der nukleinsäurebasierten Impfstoffzusammensetzung;
(ii) Lysieren der Zellkultur zum Bilden eines Zelllysats; und
(iii) Quantifizieren des in der Zellkultur exprimierten rekombinanten Proteins, für das die Vektorimpfstoffzusammensetzung oder die nukleinsäurebasierte Impfstoffzusammensetzung kodiert, unter Verwendung der Massenspektrometrie; und
wobei in dem Verfahren keine zusätzlichen Schritte zum Reinigen des rekombinanten Proteins von anderen Proteinen im Zelllysat erforderlich sind.

2. Verfahren nach Anspruch 1, wobei die Vektorimpfstoffzusammensetzung eine Adenovirus-Vektorimpfstoffkomponente umfasst, die für das rekombinante Protein kodiert, beispielsweise wobei die Vektorimpfstoffzusammensetzung für mindestens zwei rekombinante Proteine kodiert.

3. Verfahren nach Anspruch 2, wobei die Vektorimpfstoffzusammensetzung (i) eine Adenovirus-Vektorimpfstoffkomponente, die für mindestens zwei rekombinante Proteine kodiert, und/oder (ii) mehrere verschiedene Adenovirus-Vektorimpfstoffkomponenten umfasst, die für verschiedene rekombinante Proteine kodieren.

4. Verfahren nach Anspruch 1, wobei die Vektorimpfstoffzusammensetzung eine Pockenvirus-Vektorimpfstoffkomponente umfasst, die für das rekombinante Protein kodiert.

5. Verfahren nach einem vorhergehenden Anspruch, wobei:

(i) zwei oder mehr rekombinante Proteine unter Verwendung der Massenspektrometrie quantifiziert werden;

(ii) ein weiteres Protein in der Zellkultur zu mindestens 85% mit dem rekombinanten Protein identisch ist, beispielsweise wobei die Vektorimpfstoffzusammensetzung oder die nukleinsäurebasierte Impfstoffzusammensetzung ebenfalls für das weitere Protein kodiert oder das weitere Protein ein Protein aus den Zellen in der Zellkultur ist, die mit der Vektorimpfstoffzusammensetzung oder der nukleinsäurebasierten Impfstoffzusammensetzung infiziert ist; und/oder

(iii) mit dem Quantifizieren der relative oder der absolute Expressionsgrad des rekombinanten Proteins bestimmt wird.

6. Verfahren nach Anspruch 5(iii), wobei die Bestimmung:

(i) das Bestimmen des relativen Expressionsgrads des rekombinanten Proteins ist, das folgende Schritte umfasst: (a) Identifizieren von Proteinen in einer Probe mittels massenspektrometrischer Analyse; und (b) Bestimmen des relativen Expressionsgrads für jedes identifizierte Protein, darunter das rekombinante Protein, wobei gegebenenfalls die relative Bestimmung durch Bestimmen der Expression des rekombinanten Proteins bezogen auf ein Zellmarkerprotein vorgenommen wird; oder

(ii) das Bestimmen des absoluten Expressionsgrads des rekombinanten Proteins ist, das folgende Schritte umfasst: (a) Identifizieren von Proteinen in einer Probe mittels massenspektrometrischer Analyse; (b) Bestimmen des relativen Expressionsgrads für jedes identifizierte Protein, darunter das rekombinante Protein; (c) Erfassen eines proteotypischen Peptids des rekombinanten Proteins; und (d) Bestimmen des absoluten Expressionsgrads des rekombinanten Proteins durch Vergleichen der erfassten Menge des proteotypischen Peptids mit einem bekannten Standard.

7. Verfahren zum Bestimmen der Infektiosität einer Vektorimpfstoffzusammensetzung, umfassend:

(i) Infizieren einer Zellkultur mit einer Vektorimpfstoffzusammensetzung;

(ii) Lysieren der Zellkultur zum Bilden eines Zelllysats; und

(iii) Quantifizieren der intrazellulären Konzentration eines Proteins einer Vektorkomponente der Vektorimpfstoffzusammensetzung in der Zellkultur unter Verwendung der Massenspektrometrie; wobei in dem Verfahren keine zusätzlichen Schritte zum Reinigen des rekombinanten Proteins von anderen Proteinen im Zelllysat erforderlich sind.

8. Verfahren nach Anspruch 7, wobei die Vektorimpfstoffkomponente eine Adenovirus-Vektorimpfstoffkomponente ist.

9. Verfahren nach Anspruch 8, wobei die Vektorimpfstoffzusammensetzung (i) eine Adenovirus-Vektorimpfstoffkomponente, die für mindestens zwei rekombinante Proteine kodiert, und/oder (ii) mehrere verschiedene Adenovirus-Vektorimpfstoffkomponenten umfasst, die für verschiedene rekombinante Proteine kodieren.

10. Verfahren nach Anspruch 7, wobei die Vektorimpfstoffkomponente eine Pockenvirus-Vektorimpfstoffkomponente ist.

11. Verfahren nach einem der Ansprüche 7 bis 10, wobei:

(i) zwei oder mehr Proteine unter Verwendung der Massenspektrometrie quantifiziert werden;

(ii) ein weiteres Protein in der Zellkultur zu mindestens 85% mit dem Protein einer Vektorimpfstoffkomponente identisch ist, beispielsweise wobei das weitere Protein ein Protein einer weiteren Vektorimpfstoffkomponente der Vektorimpfstoffzusammensetzung ist, die Vektorimpfstoffzusammensetzung dafür kodiert oder das weitere Protein ein Protein aus den Zellen in der Zellkultur ist, die mit der Vektorimpfstoffzusammensetzung infiziert ist; und/oder

(iii) mit dem Quantifizieren der relative oder der absolute Expressionsgrad des Proteins einer Vektorimpfstoffkomponente bestimmt wird.

12. Verfahren nach Anspruch 11(iii), wobei die Bestimmung:

(i) das Bestimmen des relativen Expressionsgrads des Proteins einer Vektorimpfstoffkomponente ist, das folgende Schritte umfasst: (a) Identifizieren von Proteinen in einer Probe mittels massenspektrometrischer Analyse; und (b) Bestimmen des relativen Expressionsgrads für jedes identifizierte Protein, darunter das Protein einer Vektorimpfstoffkomponente, wobei gegebenenfalls die relative Bestimmung durch Bestimmen der Expression des rekombinanten Proteins bezogen auf ein Zellmarkerprotein vorgenommen wird; oder

(i) das Bestimmen des absoluten Expressionsgrads des Proteins einer Vektorimpfstoffkomponente ist, das folgende Schritte umfasst: (a) Identifizieren von Proteinen in einer Probe mittels massenspektrometrischer Analyse; (b) Bestimmen des relativen Expressionsgrads für jedes identifizierte Protein, darunter das Protein einer Vektorimpfstoffkomponente; (c) Erfassen eines proteotypischen Peptids des Proteins einer Vektorimpfstoffkomponente; und (d) Bestimmen des absoluten Expressionsgrads des Proteins einer Vektorimpfstoffkomponente durch Vergleichen der erfassten Menge des proteotypischen Peptids mit einem bekannten Standard.

13. Verfahren zum Prüfen der Wirksamkeit und der Infektiosität einer Vektorimpfstoffzusammensetzung, umfassend das Bestimmen der Wirksamkeit mit einem Verfahren nach einem der Ansprüche 1 bis 6 und das Bestimmen der Infektiosität mit einem Verfahren nach einem der Ansprüche 7 bis 12, gegebenenfalls bei derselben massenspektrometrischen Analyse.

14. Verfahren zum Herstellen einer Vektorimpfstoffzusammensetzung, die für ein rekombinantes Protein kodiert, oder einer nukleinsäurebasierten Impfstoffzusammensetzung, die für ein rekombinantes Protein kodiert, umfassend eine Wirksamkeitsprüfung des Impfstoffs unter Verwendung eines Verfahrens nach einem der Ansprüche 1 bis 6, Bestimmen der Infektiosität einer Vektorimpfstoffzusammensetzung unter Verwendung eines Verfahrens nach einem der Ansprüche 7 bis 12 oder Bestimmen von sowohl der Wirksamkeit als auch der Infektiosität einer Vektorimpfstoffzusammensetzung unter Verwendung des Verfahrens nach Anspruch 13.

15. Verfahren für die Herstellung von Impfstoffdosen einer Vektorimpfstoffzusammensetzung, die für ein rekombinantes Protein kodiert, oder einer nukleinsäurebasierten Impfstoffzusammensetzung, die für ein rekombinantes Protein kodiert, umfassend folgende Schritte: (i) Prüfen der relativen Wirksamkeit des Impfstoffs in einem Impfstoff-Bulk unter Verwendung eines Verfahrens nach einem der Ansprüche 1 bis 6, Bestimmen der Infektiosität eines Vektorimpfstoffs in einem Impfstoff-Bulk unter Verwendung eines Verfahrens nach einem der Ansprüche 7 bis 12 oder Bestimmen von sowohl der Wirksamkeit als auch der Infektiosität eines Vektorimpfstoffs in einem Impfstoff-Bulk unter Verwendung des Verfahrens nach Anspruch 13; und wenn die Ergebnisse von Schritt (i) für eine annehmbare relative Wirksamkeit und/oder Infektiosität sprechen, (ii) Aufteilen des Bulk-Impfstoffs auf Dosen.

## Revendications

1. Procédé d'évaluation de la puissance d'une composition de vaccin à vecteur viral codant pour une protéine recombinante ou d'une composition de vaccin à acide nucléique codant pour une protéine recombinante, le procédé comprenant :

   (i) l'infection d'une culture de cellules avec la composition de vaccin à vecteur viral ou la composition de vaccin à acide nucléique ;
   (ii) la lyse de ladite culture de cellules pour former un lysat de cellules ; et
   (iii) la quantification de la protéine recombinante codée par la composition de vaccin à vecteur viral ou la composition de vaccin à acide nucléique telle qu'exprimée dans la culture de cellules au moyen de la spectrométrie de masse ; et le procédé ne nécessitant pas d'étapes supplémentaires pour purifier la protéine recombinante à partir des autres protéines dans le lysat de cellules.

2. Procédé selon la revendication 1, dans lequel la composition de vaccin à vecteur viral comprend un composant de vaccin à vecteur adénoviral codant pour la protéine recombinante, par exemple dans lequel la composition de vaccin à vecteur viral code pour au moins deux protéines recombinantes.

3. Procédé selon la revendication 2, dans lequel la composition de vaccin à vecteur viral comprend (i) un composant de vaccin à vecteur adénoviral codant pour au moins deux protéines recombinantes et/ou (ii) des composants de vaccin à vecteur adénoviral différents multiples codant pour des protéines recombinantes différentes.

4. Procédé selon la revendication 1, dans lequel la composition de vaccin à vecteur viral comprend un composant de vaccin à vecteur de poxvirus codant pour la protéine recombinante.

5. Procédé selon l'une quelconque des revendications précédentes, dans lequel :

   (i) deux ou plus de deux protéines recombinantes sont quantifiées au moyen de la spectrométrie de masse ;
   (ii) une autre protéine dans la culture de cellules présente au moins 85 % d'identité avec la protéine recombinante, par exemple dans lequel l'autre protéine est également codée par la composition de vaccin à vecteur viral ou la composition de vaccin à acide nucléique, ou est une protéine des cellules dans la culture de cellules infectée par la composition de vaccin à vecteur viral ou la composition de vaccin à acide nucléique ; et/ou
(iii) la quantification est la détermination du taux d'expression relatif ou absolu de la protéine recombinante.

**6.** Procédé selon la revendication 5(iii), dans lequel la détermination est :

(i) la détermination du taux d'expression relatif de la protéine recombinante comprenant les étapes de : (a) identification de protéines dans un échantillon par analyse par spectrométrie de masse ; et (b) détermination du taux d'expression relatif de chaque protéine identifiée, y compris la protéine recombinante, facultativement dans lequel la détermination relative est effectuée par détermination de l'expression de la protéine recombinante par rapport à une protéine marqueur cellulaire ; ou
(ii) la détermination du taux d'expression absolu de la protéine recombinante comprenant les étapes de : (a) identification de protéines dans un échantillon par analyse par spectrométrie de masse ; (b) détermination du taux d'expression relatif de chaque protéine identifiée, y compris la protéine d'un composant de vaccin à vecteur viral ; (c) détection d'un peptide protéotypique de la protéine recombinante ; et (d) détermination du taux d'expression absolu de la protéine recombinante par comparaison de la quantité détectée du peptide protéotypique à un standard connu.

**7.** Procédé de détermination de l'infectivité d'une composition de vaccin à vecteur viral comprenant :

(i) l'infection d'une culture de cellules avec une composition de vaccin à vecteur viral ;
(ii) la lyse de ladite culture de cellules pour former un lysat de cellules ; et
(iii) la quantification du taux intracellulaire d'une protéine d'un composant de vecteur viral de la composition de vaccin à vecteur viral dans la culture de cellules au moyen de la spectrométrie de masse ; le procédé ne nécessitant pas d'étapes supplémentaires pour purifier la protéine recombinante à partir des autres protéines dans le lysat de cellules.

**8.** Procédé selon la revendication 7, dans lequel le composant de vaccin à vecteur viral est un composant de vaccin à vecteur adénoviral.

**9.** Procédé selon la revendication 8, dans lequel la composition de vaccin à vecteur viral comprend (i) un composant de vaccin à vecteur adénoviral codant pour au moins deux protéines recombinantes et/ou (ii) des composants de vaccin à vecteur adénoviral différents multiples codant pour des protéines recombinantes différentes.

**10.** Procédé selon la revendication 7, dans lequel le composant de vaccin à vecteur viral est un composant de vaccin à vecteur de poxvirus.

**11.** Procédé selon l'une quelconque des revendications 7 à 10, dans lequel :

(i) deux ou plus de deux protéines sont quantifiées au moyen de la spectrométrie de masse ;
(ii) une autre protéine dans la culture de cellules présente au moins 85 % d'identité avec la protéine d'un composant de vaccin à vecteur viral, par exemple dans lequel l'autre protéine est une protéine d'un autre composant de vaccin à vecteur viral de la composition de vaccin à vecteur viral, est codée par la composition de vaccin à vecteur viral, ou est une protéine des cellules dans la culture de cellules infectée par la composition de vaccin à vecteur viral ; et/ou
(iii) la quantification est la détermination du taux d'expression relatif ou absolu de la protéine d'un composant de vaccin à vecteur viral.

**12.** Procédé selon la revendication 11(iii), dans lequel la détermination est :

(i) la détermination du taux d'expression relatif de la protéine d'un composant de vaccin à vecteur viral comprenant les étapes de : (a) identification de protéines dans un échantillon par analyse par spectrométrie de masse ; et (b) détermination du taux d'expression relatif de chaque protéine identifiée, y compris la protéine d'un composant de vaccin à vecteur viral, facultativement dans lequel la détermination relative est effectuée par détermination de l'expression de la protéine recombinante par rapport à une protéine marqueur cellulaire ; ou
(ii) la détermination du taux d'expression absolu de la protéine d'un composant de vaccin à vecteur viral comprenant les étapes de : (a) identification de protéines dans un échantillon par analyse par spectrométrie de masse ; (b) détermination du taux d'expression relatif de chaque protéine identifiée, y compris la protéine d'un composant de vaccin à vecteur viral ; (c) détection d'un peptide protéotypique de la protéine d'un composant de vaccin à vecteur viral ; et (d) détermination du taux d'expression absolu de la protéine d'un composant de vaccin à vecteur viral par comparaison de la quantité détectée du peptide protéotypique à un standard connu.

13. Procédé d'évaluation de la puissance et de l'infectivité d'une composition de vaccin à vecteur viral, comprenant la détermination de la puissance par un procédé selon l'une quelconque des revendications 1 à 6 et la détermination de l'infectivité par un procédé selon l'une quelconque des revendications 7 à 12, facultativement dans la même analyse par spectrométrie de masse.

14. Procédé de fabrication d'une composition de vaccin à vecteur viral codant pour une protéine recombinante ou d'une composition de vaccin à acide nucléique codant pour une protéine recombinante, comprenant l'évaluation de la puissance du vaccin au moyen d'un procédé selon l'une quelconque des revendications 1 à 6, la détermination de l'infectivité d'une composition de vaccin à vecteur viral au moyen d'un procédé selon l'une quelconque des revendications 7 à 12, ou la détermination de la puissance et de l'infectivité d'une composition de vaccin à vecteur viral au moyen du procédé selon la revendication 13.

15. Procédé de fabrication de doses de vaccin d'une composition de vaccin à vecteur viral codant pour une protéine recombinante ou une composition de vaccin à acide nucléique codant pour une protéine recombinante, comprenant les étapes de : (i) évaluation de la puissance relative du vaccin dans un vrac de vaccin au moyen d'un procédé selon l'une quelconque des revendications 1 à 6, détermination de l'infectivité d'un vaccin à vecteur viral dans un vrac de vaccin au moyen d'un procédé selon l'une quelconque des revendications 7 à 12, ou détermination à la fois de la puissance et de l'infectivité d'un vaccin à vecteur viral dans un vrac de vaccin au moyen d'un procédé selon la revendication 13 ; et, si les résultats de l'étape (i) indiquent une puissance et/ou infectivité relative acceptable, (ii) distribution du vaccin en vrac dans des doses.

*Figure 1*

*Figure 2*

*Figure 3*

*Figure 4*

*Figure 5*

*Figure 6*

*Figure 7*

*Figure 8*

*Figure 9*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012082918 A
- US 5385839 A
- US 5559099 A
- US 5837511 A
- US 5846782 A
- US 5851806 A
- US 5994106 A
- US 5994128 A
- US 5965541 A
- US 5981225 A
- US 6040174 A
- US 6020191 A
- US 6113913 A
- US 5494807 A
- US 5185146 A
- US 5093258 A
- US 5445953 A
- EP 0308220 A1
- US 5830463 A
- WO 2009026183 A
- WO 2010096561 A
- WO 2006120034 A
- WO 0222080 A
- WO 0102607 A
- WO 2004037294 A
- JP 3022979 A
- JP 2227075 A
- WO 9710354 A
- WO 2014134561 A
- EP 1118860 A
- EP 15159717

### Non-patent literature cited in the description

- **HAVE et al.** *Biologicals,* 2012, vol. 40 (1), 84-7
- **REZAPKIN et al.** *Biologicals,* 2005, vol. 33 (1), 17-27
- **BADGER et al.** *Vaccine,* 2011, vol. 29 (39), 6728-35
- **WEINER et al.** *Molecular Therapy,* vol. 21, 506-508
- **BAROUCH et al.** *Nat Med,* 2010, vol. 16 (3), 319-323
- **HU et al.** *J Mass Spectrom.,* April 2005, vol. 40 (4), 430-43
- **ENG et al.** *J Am Soc Mass Spectrom,* 1994, vol. 5 (11), 976-989
- **PERKINS et al.** *Electrophoresis,* 1999, vol. 20 (18), 3551-67
- **KÄLL et al.** *Nature Methods,* 2007, vol. 4, 923-925
- **PENG et al.** *Nat Methods,* 2012, vol. 9 (6), 524-5
- **SCHWANHÄUSSER et al.** *Nature,* 2011, vol. 473 (7347), 337-42
- **ERRATUM.** *Nature,* 07 March 2013, vol. 495 (7439), 126-7
- **SILVA et al.** *Anal Chem,* 2005, vol. 77 (7), 2187-200
- **AYE et al.** *Mol Biosyst,* 2010, vol. 6 (10), 1917-27
- **MALMSTRÖM et al.** *Nature,* 2009, vol. 460 (7256), 762-5
- **SILVA et al.** *Mol Cell Proteomics,* 2006, vol. 5, 144-156
- **GROSSMANN et al.** *J Proteomics,* 2010, vol. 73, 1740-1746
- **KONDRAT et al.** *Anal Chem,* 1978, vol. 50 (14), 2017-21
- **GERGOV et al.** *J. Chromatogr., B: Anal. Technol. Biomed. Life. Sci.,* 2003, vol. 795 (1), 41-53
- **KUZYK et al.** *Mol. Cell. Proteomics,* 2009, vol. 8 (8), 1860-1877
- **SEPPALA et al.** *J. proteome,* 2011, vol. 10, 2113-2122
- **KIYONAMI et al.** *Mol.Cell. Proteomics,* 2011, vol. 10 (2
- **GERBER et al.** *PNAS,* 2003, vol. 100 (12), 6940-45
- **BRUN et al.** *J. Proteomics,* 2009, vol. 72 (5), 740-49
- **SHIVER et al.** *Nature,* 2002, vol. 415 (6869), 331-5
- **HILL et al.** *Hum Vaccine,* vol. 6 (1), 78-83
- **SULLIVAN et al.** *Nature,* 2000, vol. 408 (6812), 605-9
- **SANTRA et al.** *Vaccine,* 2009, vol. 27 (42), 5837-45
- Adenoviridae and their Replication,. **THOMAS SHENK.** Adenoviruses
- Virology. Raven Press, Ltd, 1996

- **SAMBROOK et al.** Molecular Cloning, a Laboratory Manual. Cold Spring Harbor Press, 1989
- Recombinant DNA. **WATSON et al.** Scientific American Books. 1992
- **AUSUBEL et al.** Current Protocols in Molecular Biology. Wiley Interscience Publishers, 1995
- **WANG et al.** *Virology,* 2004, vol. 330, 375-83
- **MACKETT et al.** *Proc. Natl. Acad. Sci. USA,* 1982, vol. 79 (741), 5-7419
- **MEDINA et al.** *Vaccine,* 2001, vol. 19 (13-14), 1573-1580
- **GARMORY et al.** *J Drug Target,* 2003, vol. 11 (8-10), 471-479
- **WEISS ; KRUSCH.** *Biol. Chem.,* 2001, vol. 382, 533-41
- **DARJI et al.** *FEMS Immunol and Medical Microbiology,* 2000, vol. 27, 341-9
- **STUBBS et al.** *Nature Medicine,* 2001, vol. 7, 625-29
- **SULLIVAN et al.** *Nature,* 2003, vol. 424 (6949), 681-684
- **SULLIVAN et al.** *PLoS Med,* 2006, vol. 3 (6), e177
- **GEISBERT et al.** *J Virol,* 2011, vol. 85, 4222-4233
- **ZHOU et al.** *Mol Ther,* 2010, vol. 18, 2182-9
- **HU et al.** *Virus Res,* 2011, vol. 155, 156-62
- **VEMULA ; MITTAL.** *Expert Opin Biol Ther,* 2010, vol. 10, 1469-87
- **ZHOU et al.** *Mol Ther,* 2006, vol. 14, 662-672
- **YANG ; ZUBAREV.** *Electrophoresis,* 2011, vol. 31, 1764-1772
- **DIEPOLD et al.** *PLoS One,* 2012, vol. 7, e30295
- **CORRADINI et al.** *Mol Cell Proteomics,* 2014, vol. 13, 2004-2016
- Simultaneous quantification of hemagglutinin and neuramidase of influenza virus using isoptope dilution mass spectroscopy. **WILLIAMS T L et al.** Vaccine. Elsevier LTD, 23 March 2012, vol. 30, 2475-2482
- **MELKAMU GETIE-KEBTIE et al.** Label-free mass spectrometry-based quantification of hemagglutinin and neuraminidase in influenza virus preparation and vaccines. *Influenza and other respiratory viruses,* 03 July 2013, vol. 7 (4), 521-530
- **WANDA I. SANTANA et al.** Quantification of Viral Proteins of the Avian H7 subtype of Influenza Virus: An Isotope Dilution Mass Spectrometry Method Applicable for Producing more Rapid Vaccines in the case of an Influenza Pandemic. *Analytical Chemistry,* 01 April 2014, vol. 86 (9), 4088-4095